(19) Europäisches Patentamt
European Patent Office
Office européen des brevets

(11) Publication number: 0 194 403 A1

(12) **EUROPEAN PATENT APPLICATION**

(21) Application number: 86100018.0

(22) Date of filing: 02.01.86

(51) Int. Cl.[4]: C 07 C 103/46
C 07 C 103/66, C 07 D 207/16
C 07 D 295/18, C 07 C 149/243
A 01 N 39/02, A 01 N 43/36

**A request for correction of description and claims has been filed pursuant to Rule 88 EPC. A decision on the request will be taken during the proceedings before the Examining Division.**

(30) Priority: 14.01.85 JP 3251/85

(43) Date of publication of application: 17.09.86 Bulletin 86/38

(84) Designated Contracting States: CH DE FR GB LI

(71) Applicant: TEIJIN LIMITED
11 Minami Honmachi 1-chome Higashi-ku
Osaka-shi Osaka-fu(JP)

(72) Inventor: Azuma, Shizuo
28-2, Ozu-cho 1-chome
Iwakuni-shi Yamaguchi-ken(JP)

(72) Inventor: Hiramatsu, Toshiyuki
28-3, Ozu-cho 1-chome
Iwakuni-shi Yamaguchi-ken(JP)

(72) Inventor: Yamaji, Teizo
5-10, Yamate-cho 4-chome
Iwakuni-shi Yamaguchi-ken(JP)

(72) Inventor: Ichikawa, Yataro
11-7, Kotesashi-cho 2-chome
Tokorozawa-shi Saitama-ken(JP)

(74) Representative: Kraus, Walter, Dr. et al,
Patentanwälte Kraus, Weisert & Partner
Thomas-Wimmer-Ring 15
D-8000 München 22(DE)

(54) **Novel amide compound and herbicide comprising it.**

(57) An amide of ω – (2,4–disubstituted phenoxy) aliphatic carboxylic acid and ω-aminoaliphatic carboxylic acid represented by the general formula (1).

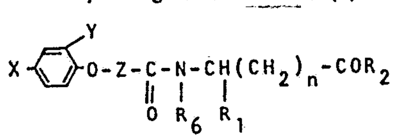

wherein:
X is Cl or $CF_3$
Y is Cl or $CH_3$
Z is a divalent hydrocarbon radical having 1 to 4 carbon atoms
$R_6$ is hydrogen or a hydrocarbon radical
$R_1$ is hydrogen n or an eventually substituted hydrocarbon radical or forms with $R_6$ a $CH_2{-}CH_2{-}CH_2$ - group
n is 0,1 or 2
$R_2$ is an alkoxy or amino group

The amide is used for controlling weeds by applying a herbicidally sufficient amount of the amide to the seeds, stalks, leaves or roots of plants to be controlled or to the locus where the plants are growing or the growth of said plants is anticipated.

EP 0 194 403 A1

Croydon Printing Company Ltd.

This invention relates to a novel amide compound formed between a phenoxy aliphatic carboxylic acid and an aminocarboxylic acid and a herbicide comprising the amide as an active ingredient.

It has been known that phenoxy aliphatic carboxylic acids have herbicidal activity. R. Pokorny first synthesized 2,4-dichlorophenoxyacetic acid [J. Amer. Chem. Soc., Vol. 63, page 1768 (1941)]. Later, its activity against plants was discovered, and its selective herbicidal activity was recognized [Nature, Vol. 155, page 498 (1945)].

The latter literature reference discloses that 2,4-dichlorophenoxyacetic acid (sometimes referred to simply as 2,4-D hereinafter) inhibits the growth of the roots of sugar beet and clover while having little effects of inhibiting the growth of wheat, and that in view of its different toxicity susceptibilities on different types of plant, 2,4-D is of practical importance.

The synthesis of 2,4-D stimulated development of many phenoxy aliphatic carboxylic acids having similar skeletons and their derivatives, and some of them came into practical acceptance. The development of these phenoxy aliphatic carboxylic acids and their derivatives has been directed to the changing or modification of (a) the phenoxy group, (b) the aliphatic group, and (c) the carboxyl group. The changing or modification of the phenoxy group (a) includes, for example, the changing of the positions of bonding chlorine atoms or the number of chlorine atoms, the introduction of a methyl group or a chlorophenoxy group or a pyridyloxy group substituted by chloro or trifluoromethyl. The changing or modification of the aliphatic group (b) includes, for example, the conversion of the methylene group into an ethylene, ethylidene or trimethylene group. The changing or modification of the carboxyl group (c) includes, for example, the conversion of the carboxyl group into the group $-OSO_3Na$, the group -OH, the group $-COSC_2H_5$ or the amide group.

These prior research and delopment efforts will be specifically discussed below.

Nature, 155, page 497 (1954) describes that beta-indoleacetic acid or alpha-naphthylacetic acid in a dose of 275 g/10a does not wither oats, wheat, barley and rye, but withers charlock, yarrow and plantain. It further discloses that 2,4-D and 4-chloro-2-methylphenoxyacetic acid have stronger activity than alpha-naphthylacetic acid, and that 4-chloro-2-methylphenoxyacetic acid inhibits the germination and growth of corn buttercup, fat hen, corn marigold and field poppy and in a dose of 110 g/10a withers charlock, corn buttercup and pennycress without harming cereals and oats.

Nature, 155, page 500 (1945) discloses that 4-chloro-2-methylphenoxyacetic acid withers buttercup, horsetail and creeping thistle, but not maize, stinging nettle and bracken.

Botanical Gazette, 108, page 301 (1947) describes the effects of various aryloxyacetic acids including 2,4-D by foliar treatment. Blue rose rice, barley, wheat, sugar beets, corn, buckwheat, clover, rape, radish and bean are used as crops, but the reference does not describe amides of aryloxyacetic acids.

Agricultural and Food Chemistry, 4, page 690 (1956) describes the physiological activities of amides of 2,4-dichlorophenoxy-2-propionic acid with alanine, aspartic acid, leucine, methionine, phenylalanine and threonine on useful crops such as corn, barley, cucumber, sunflower and soybean. Specifically, it states that while the alanine amide, leucine amide and threonine amide almost completely withered broad-leaved crops such as soybean, sunflowr and cucumber and inhibited growth of narrow-leaved plants such

as barley and corn to some extent, the aspartic acid amide, methionine amide and phenylalanine amide showed little or no effect against broad-leaved or narrow-leaved crops.

Agricultural and Food Chemistry, 7, page 118 (1959) describes the effects of phenoxyacetic acids such as 2,4-D or phenoxypropionic acids such as 2,4-dichlorophenoxypropionic acid and the p-toluenesulfonamides, p-aminobenzamides and p-aminosalicylamides of these parent compounds on useful crops such as corn, barley, cucumber, sunflower and soybean in foliar treatment. It specifically discloses that the activities of the p-aminobenzamides and the p-aminosalicylamides are equivalent to, or slightly lower than, those of the parent compounds.

Journal of Organic Chemistry, 17, page 891 (1952) gives a disclosure about the synthesis of amides of 2,4-D with L- or DL-alanine, L-, D- or DL-aspartic acid, L- or DL-glutamic acid, glycine, DL-histidine, L- or DL-leucine, DL-iso-leucine, L-, D- or DL-methionine, L-, D- or DL-phenylalanine, L- or DL-proline, DL-serine, DL-threonine, DL-tryptophan, DL-valine, L-cystine, L- or DL-lysine, and DL-tyrosine methyl ester. This paper reports the results of examining the growth controlling effect of these amides on tomato, and states that the D-aspartic acid amide, the D-methionine amide and the D-phenylalanine amide failed to induce formative changes in tomato plants, but the other amides induced formative changes in tomato plants.

Weed Science, 3, page 28 (1954) deals with synthetic plant growth modifiers, and discloses amides of 2-methyl-4-chloroacetic acid and L-, D- or DL-alanine, L-, D- or DL-leucine, L-, D- or DL-aspartic acid, L-, D- or DL-methionine, L-, D- or DL-phenylalanine and L-, D- or DL-threonine. This paper shows the growth-modifying properties of these compounds on pinto bean, black valentine bean, sunflower, cucumber, barley and corn.

Journal of Organic Chemistry, 25, page 143 (1960) gives a disclosure about the synthesis of amides of 2,4-D

with D-alanine, beta-alanine, D-, L- or DL-asparagine, D-glutamic acid, glycylglycine, D-leucine, D- or L-isoleucine, D- or L-serine, D- or L-threonine, D- or L-tryptophan, D- or L-valine and D- or DL-cystine. This paper discusses the possibility of using these amides as plant growth regulators or bioactive formulating agents, and shows the selective activity of the amide of 2,4-D and D-asparagine whereby in a dose of 1 pound per acre, it kills pigweed and mustard but does not affect corn and gladiolus.

An Evaluation of Several Chemicals for Their Herbicidal Properties <u>1957 Field Results</u> by W. A. Gentner and W. C. Shaw, United States Department of Agriculture, Agricultural Research Service, Crop Research Division, Plant Industry Station, Beltsville, Maryland, January 1958 shows data on the degree of injury of $N^2$-[(2,4-dichlorophenoxy)acetyl]-D-asparagine to lima beans, cucumber, cotton, soybeans, sugar beats, snapbeans, lespedeza, ladino clover, alfalfa, red clover, corn, peanuts, buckwheat, sorghum, flax, gladiolus, sudan grass, squash, oats, cowpeas and Bird-foot trefoil.

An Evaluation of Several Chemicals for Their Herbicidal Properties <u>1958 Field Results</u> by W. A. Gentner and W. C. Shaw, United States Department of Agriculture, Agricultural Research Service, Crop Research Division, Plant Industry Station, Beltsville, Maryland, January 1959 gives post emergency data showing the injury ratings of amides of 2,4-dichlorophenoxypropionic acid with L-methionine, DL-phenylalanine and L-leucine on crops such as soybeans, cucumber, cotton, beets, corn, peanuts, barley and clover and weeds such as crabgrass, lambsquarters, ryegrass and mustard. This document discloses that the aforesaid amides do not have selectivity for lambsquarters as a broadleaf weed and crabgrass as a narrowleaf weed.

This publication also describes that by preemergence spray, the above three types of amides are relatively inactive against the aforesaid crops and weeds.

French Patent Specification No. 1,544,786 discloses that amides of p-halogenophenoxyacetic acids with glycine, alanine, leucine, isoleucine, aspartic acid, arginine, histidine, lysine, phenylalanine, serine and methionine have herbicidal activity.

U. S. Patent No. 2,734,816 describes D-amino acids acetylated with nuclearly chlorinated phenoxyacetic acids such as 2,4-dichlorophenoxyacetic acid and discloses that these compounds have a hormonal effect of increasing the size of fruits or promoting the growth of tomato. The patent shows that the D-amino acid derivatives have the aforesaid effect in concentrations of 4 to 30 ppm.

U. S. Patent No. 3,557,209 discloses compounds of the following formula

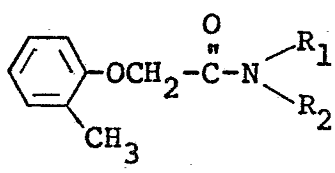

wherein $R_1$ is selected from the group consisting of propargyl, haloalkyl of up to 5 carbon atoms, carboxyalkyl of up to 3 carbon atoms and lower alkoxy alkyl; and $R_2$ is selected from the group consisting of hydrogen, propargyl, haloalkyl of up to 5 carbon atoms, carboxyalklyl of up to 3 carbon atoms and lower alkoxyalkyl,

and states that the above compounds can be applied as herbicides.

It is an object of this invention to provide a novel amide compound formed between a phonoxy aliphatic carboxylic acid and an aminocarboxylic acid derivative.

Another object of this invention is to provide a novel amide compound having herbicidal activity.

Another object of this invention is to provide a herbicide comprising as an active ingredient an amide of a

phenoxy aliphatic carboxylic acid with an amino acid derivative.

Another object of this invention is to provide a herbicide which has a selectivity of eradicating weeds without substantially inhibiting the growth of useful cereal crops such as rice and corn.

Another object of this invention is to provide a herbicide which can inhibit the growth of, or eradicate, the undesired plants in low dosages per unit area, for example, in doses of less than 1kg per hectare.

Another object of this invention is to provide a selective herbicide which acts on buds after germination and withers, or inhibits the growth of, not only broad-leaved weeds but also some narrow-leaved weeds, and therefore in an area where useful crops such as rice and corn and noxious weeds grow together, creates a condition in which the useful crops can grow more than the noxious weeds.

Another object of this invention is to provide a selective herbicide which can wither, or inhibit the growth of, weeds by foliar application, and also inhibit the germination of weeds without substantially inhibiting the germination of useful crops by preemergence soil application.

Another object of this invention is to provide a selective herbicide having low animal and fish toxicity.

An additional object of this invention is to provide a selective herbicide which can be easily produced at relatively low costs.

Other objects and advantages of this invention will become apparent from the following description.

Investigations of the present inventors have shown that these objects of the invention are achieved by a compound represented by the following general formula (1)

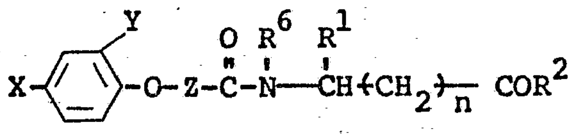 ... (1)

wherein

X is Cl or $CF_3$,

Y is Cl or $CH_3$,

Z represents a divalent saturated or unsaturated aliphatic hydrocarbon group having not more than 4 carbon atoms,

$R^1$ represents a group selected from the class consisting of a hydrogen atom, saturated aliphatic hydrocarbon groups having not more than 4 carbon atoms, a phenyl group, a benzyl group, a beta-methylthioethyl group, a hydroxymethyl group, an alpha-hydroxyethyl group, a hydroxyphenylmethyl group, a mercaptomethyl group and a benzylthiomethyl group,

$R^2$ represents the group $OR^3$ or the group

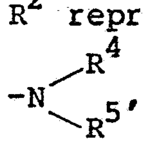

$R^3$ represents an aliphatic hydrocarbon group having not more than 15 carbon atoms which may be interrupted by an oxygen atom and may have a substituent, a phenyl group which may have a substituent or an ammonium cation,

$R^4$ and $R^5$ are identical or different and each represents a hydrogen atom, a saturated aliphatic hydrocarbon group having not more than 6 carbon atoms, or an alicyclic hydrocarbon group having 5 to 7 carbon atoms, or $R^4$ and $R^5$, taken together with the nitrogen atom to which they are bonded, may form a 5- to 7-membered ring which may further contain a hetero atom,

$R^6$ represents a hydrogen atom or a saturated aliphatic hydrocarbon having not more than 4 carbon atoms or, taken together with $R^1$, may form $-CH_2CH_2CH_2-$ or $-CH_2CH_2\overset{OH}{\overset{|}{C}}H-$, and n is 0, 1 or 2.

In general formula (1), X is Cl or $-CF_3$, and Y is Cl or $-CH_3$. In order to achieve the objects of this invention, X and Y need to be the above atom or groups. If one or both of X and Y are hydrogen atoms or groups or atoms other than Cl, $CF_3$ and $CH_3$, the herbicide has lower herbicidal activity and selectivity. Preferably, both of X and Y are Cl, or X is Cl and Y is $-CH_3$.

Z is a divalent saturated or unsaturated aliphatic hydrocarbon group having not more than 4 carbon atoms, which may be linear or branched. Preferred examples are $-CH_2-$, $-\overset{CH_3}{\overset{|}{C}}H-$, $-CH_2CH_2-$, $-\overset{CH_3}{\overset{|}{C}}HCH_2-$, $-CH_2CH_2CH_2-$, $-\overset{CH_3}{\overset{|}{C}}HCH_2CH_2-$, $-CH_2\overset{CH_3}{\overset{|}{C}}HCH_2-$, $-\overset{CH_3}{\overset{|}{C}}H-\overset{CH_3}{\overset{|}{C}}H-$, $-\underset{CH_3}{\underset{|}{\overset{CH_3}{\overset{|}{C}}}}-CH_2-$, $-CH=CH-$, $-\overset{CH_3}{\overset{|}{C}}=CH-$, $-\overset{CH_2}{\overset{\|}{C}}-$, $-\overset{CH_2}{\overset{\|}{C}}-CH_2-$, $-CH_2-CH=CH-$ and $-CH=CH-CH=CH-$.

Of these, $-CH_2-$, $-\overset{CH_3}{\overset{|}{C}}H-$ and $-CH_2CH_2CH_2$ are especially preferred. Above all, when X and Y are both Cl, Z is preferably $-CH_2-$. When X is Cl and Y is $-CH_3$, Z is preferably an alkylene group having 1 to 3 carbon atoms such as $-CH_2-$, $-\overset{CH_3}{\overset{|}{C}}H-$ or $-CH_2CH_2CH_2-$.

In general formula (1), $R^1$ is a hydrogen atom, a saturated hydrocarbon group having not more than 4 carbon atoms, phenyl (-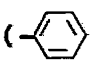), benzyl ($-CH_2-$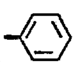), beta-methylthioethyl ($-CH_2CH_2SCH_3$), hydroxymethyl ($-CH_2OH$), alpha-

hydroxyethyl ($-\overset{CH_3}{CHOH}$), hydroxyphenylmethyl ($-CH_2-$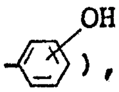), mercaptomethyl ($-CH_2SH$), or benzylthiomethyl ($-CH_2SCH_2-$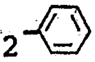).

The saturated aliphatic hydrocarbon group having not more than 4 carbon atoms may be linear or branched, and specifically includes methyl, ethyl, n-propyl, iso-propyl, n-butyl, iso-butyl and tert-butyl.

$R^1$ is preferably hydrogen, methyl, ethyl, n-propyl, iso-propyl, n-butyl, iso-butyl, sec-butyl, t-butyl, phenyl, benzyl or beta-methylthioethyl.

$R^2$ is $-OR^3$ or 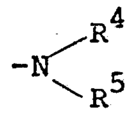, preferably the former.

$R^3$ is an aliphatic hydrocarbon group having not more than 15 carbon atoms which may be interrupted by an oxygen atom and may have a substituent, a phenyl group which may have a substituent, or an ammonium cation (in which case the COO group to which the ammonium cation is bonded is naturally an anion).

Examples of the aliphatic hydrocarbon group having not more than 15 carbon atoms are methyl, ethyl, n-propyl, iso-propyl, n-butyl, iso-butyl, sec-butyl, tert-butyl, n-pentyl, iso-pentyl, n-hexyl, n-octyl, n-nonyl, n-decyl, n-undecyl, n-dodecyl, n-tridecyl, n-tetradecyl, and n-pentadecyl. The aliphatic hydrocarbon group having not more than 15 carbon atoms may be linear or branched as indicated above. The aliphatic hydrocarbon group interrupted by an oxygen atom may, for example, be $-CH_2CH_2OCH_3$, $-CH_2CH_2OCH_2CH_3$, $-CH_2CH_2OCH_2CH_2OCH_3$, or $-CH_2CH_2OCH_2CH_2OCH_2CH_3$.

The substituent on the above aliphatic hydrocarbon group may, for example, be a phenyl group, a tolyl group, a hydroxyl group, a halogen atom, etc.

The substituent on the above phenyl group may, for example, be a halogen atom such as fluorine, chlorine, or an alkyl group having 1 to 5 carbon atoms.

Preferably, $R^3$ is an unsubstituted aliphatic hydrocarbon group having not more than 15 carbon atoms, an aliphatic hydrocarbon group having not more than 15 carbon atoms and a phenyl or hydroxyl group as the substituent, an unsubstituted aliphatic hydrocarbon group having not more than 10 carbon atoms and being interrupted by an oxygen atom, a phenyl group or a phenyl group substituted by an alkyl group having 1 to 5 carbon atoms.

More preferably, $R^3$ is an alkyl group having 1 to 6 carbon atoms.

The ammonium cation is represented, for example, by the following formula

$$H\overset{+}{N}R_3$$

wherein the three R's are identical or different and each represents a hydrogen atom, alkyl having 1 to 6 carbon atoms, cyclopentyl, cyclohexyl, or phenyl, or two R's may be bonded to each other to form tetramethylene, pentamethylene or ethyleneoxyethylene. The $C_1$-$C_6$ alkyl may be linear or branched, and includes, for example, methyl, ethyl, n-propyl, iso-propyl, n-butyl, iso-butyl, sec-butyl, n-pentyl, and n-hexyl.

In the group 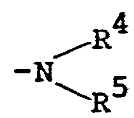, $R^4$ and $R^5$ are identical or different and each represents a hydrogen atom, a saturated aliphatic hydrocarbon group having not more than 6 carbon atoms, or an alicyclic hydrocarbon group having 5 to 7 carbon atoms. Examples of the saturated aliphatic hydrocarbon group having not more than 6 carbon atoms will be clear from the examples of $R^3$ given above. The alicyclic hydrocarbon groups having 5 to 7 carbon atoms are cyclopentyl, cyclohexyl and cycloheptyl. Preferably, each of $R^4$ and $R^5$ is hydrogen, methyl, ethyl, cyclopentyl, cyclohexyl or cycloheptyl.

Alternatively, $R^4$ and $R^5$, taken together with the nitrogen atom to which they are bonded, may form a 5- to 7-membered ring which may further contain a hetero atom.

$R^4$ and $R^5$, taken together, may form $-(CH_2)_4-$, $-(CH_2)_5-$, $-(CH_2)_2O(CH_2)_2$ or $-(CH_2)_2NH(CH_2)_2$.

$R^6$ is a hydrogen atom, or a saturated aliphatic hydrocarbon group having not more than 4 carbon atoms. Or $R^6$ may be taken together with $R^1$ to form $-CH_2CH_2CH_2-$ or $-CH_2CH_2\overset{OH}{C}H-$. Examples of the saturated aliphatic hydrocarbon group having not more than 4 carbon atoms will be clear from the examples of $R^1$.

Preferably, $R^6$ is hydrogen, methyl, ethyl, n-propyl, iso-propyl, n-butyl, iso-butyl, or tert-butyl, or taken together with $R^1$, forms $-CH_2CH_2CH_2-$ or $-CH_2CH_2\overset{OH}{C}H-$.

n is 0, 1 or 2. It should be understood that when n is 0, $-\overset{R^1}{C}H-$ is directly bonded to $-COR^2$. Preferably, n is 0.

In general formula (1), 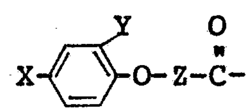 on the left side is the skeletal portion of a phenoxy aliphatic carboxylic acid, and the $-\overset{R^6}{N}-\overset{R^1}{C}H(CH_2)_nCOR^2$ on the right side is the skeletal portion of an aminocarboxylic acid derivative. The aminocarboxylic acid of the derivative may be an alpha-, beta- or gamma-amino acid corresponding to the value of n. Examples of such amino acids include alpha-amino acids such as glycine, alanine, valine, isoleucine, leucine, cysteine, methionine, phenylalanine, phenylglycine, serine, threonine and proline; and beta- or gamma-amino acids such as

$NH_2CH_2CH_2COOH$, $NH_2(CH_2)_3COOH$, $NH_2\overset{CH_3}{C}HCH_2COOH$,

$NH_2\overset{C_2H_5}{C}HCH_2COOH$, $NH_2\overset{C_3H_7}{C}HCH_2COOH$, $NH_2\overset{CH(CH_3)_2}{C}HCH_2COOH$,

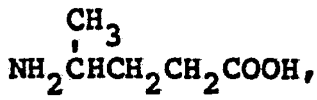, 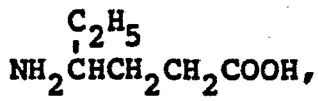,

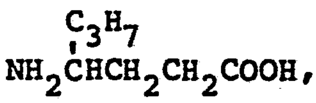, and 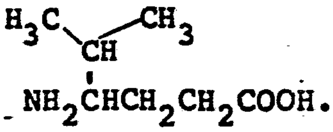.

Among the above amino acids, glycine, alanine, beta-alanine, valine, isoleucine, leucine, methionine, phenylalanine and phenylglycine are especially preferred for the amino acid derivatives of this invention.

Specific examples of the compounds of general formula (1) are shown below for facilitating an understanding of this invention without any intention of limiting the invention thereby.

(1) 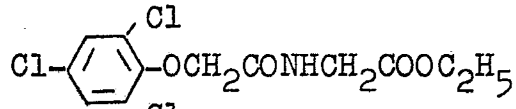

(2) 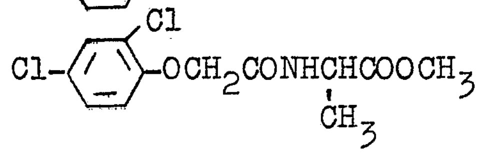

(3) 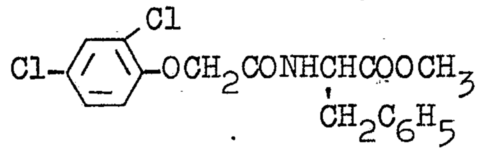

(4) 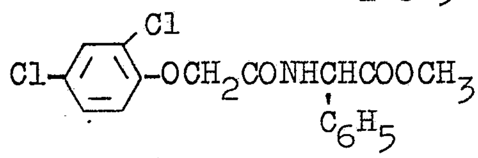

(5) 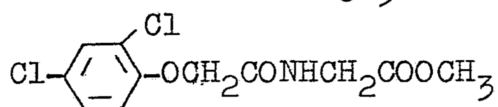

(6) 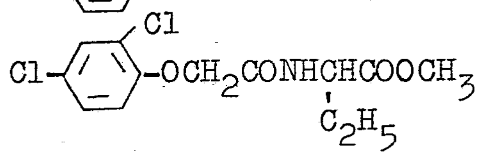

(7) 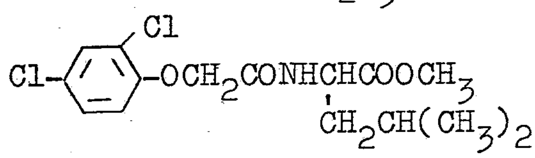

(8) 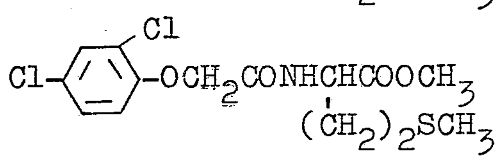

(9) 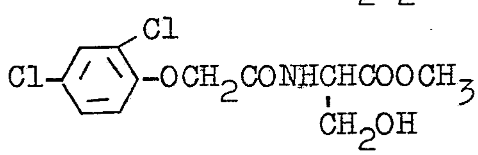

(10) 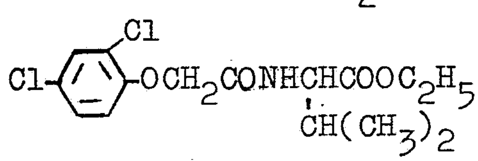

(11) 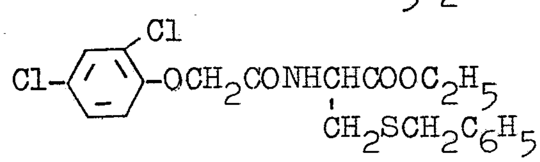

(12) 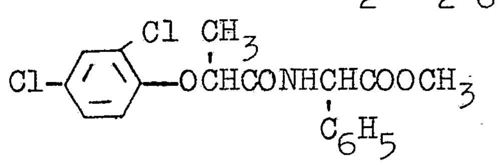

(13) 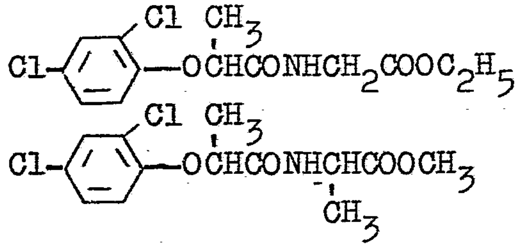

(14)

(15) 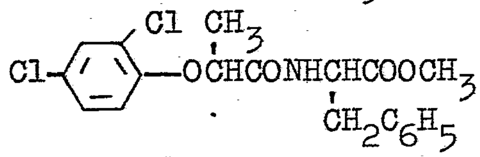

(16) 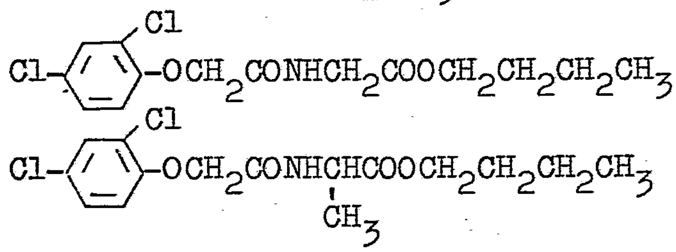

(17)

(18) 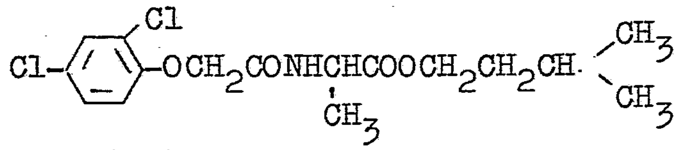

(19) 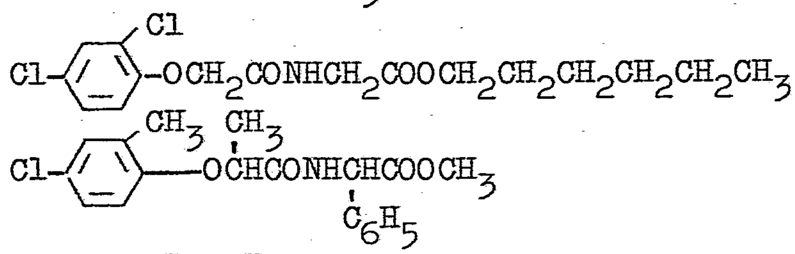

(20)

(21) 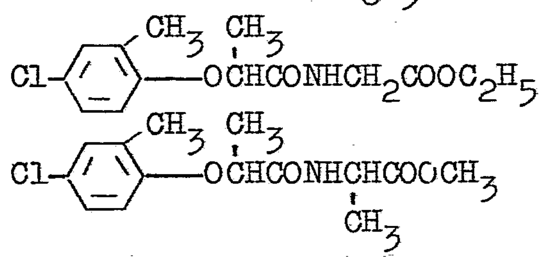

(22)

(23) 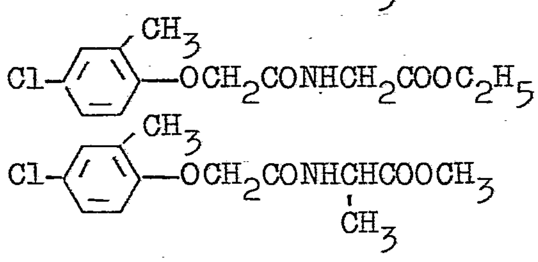

(24)

(25) 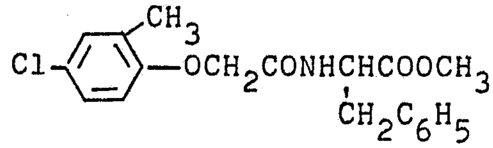

(26) 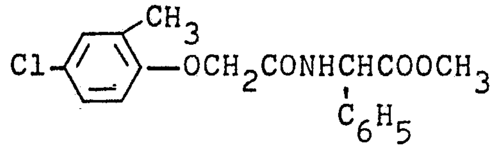

(27) 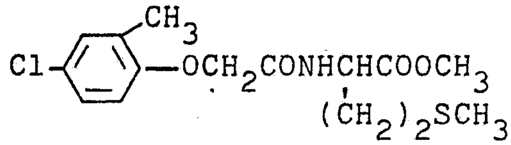

(28) 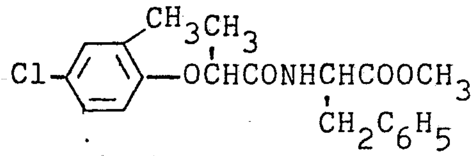

(29) 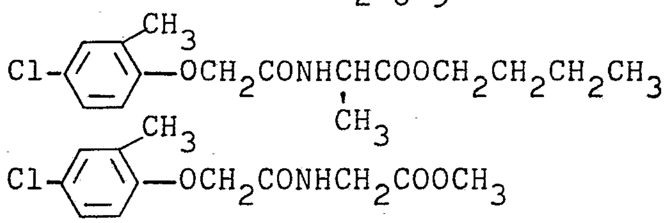

(30)

(31) 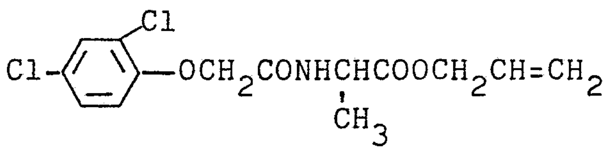

(32) 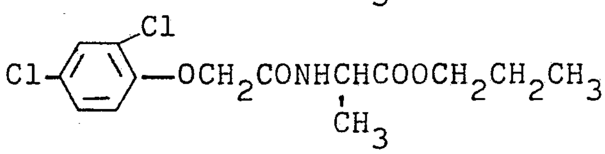

(33) 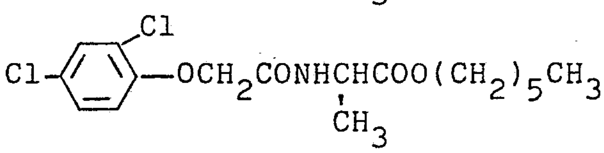

(34) 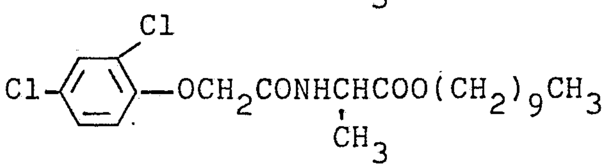

(35) 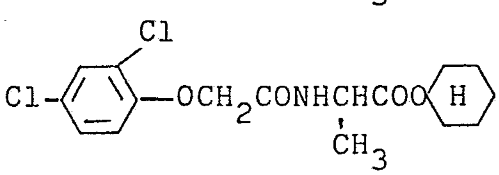

(36) 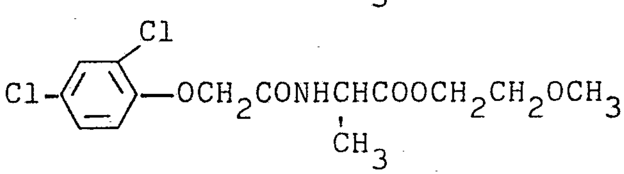

(37) 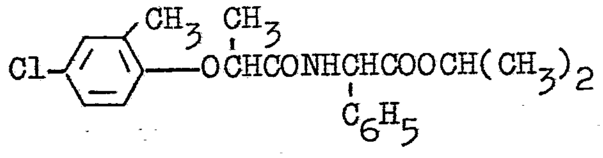

(38) 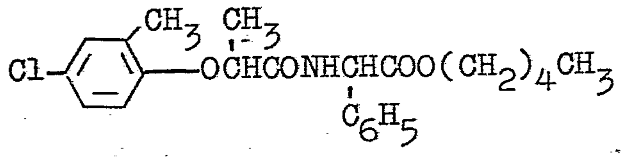

(39) 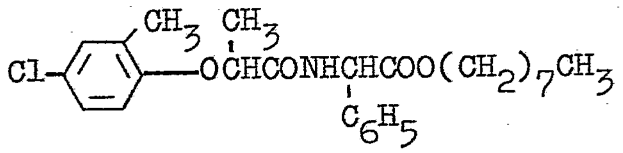

(40) 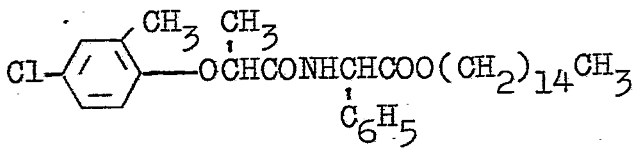

(41) 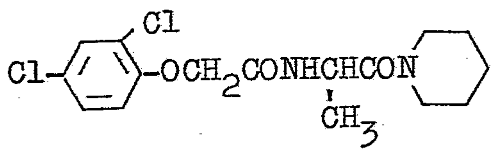

(42) 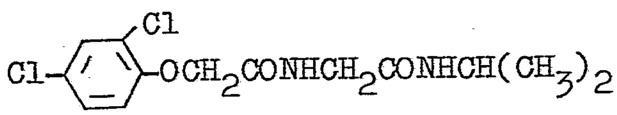

(43) 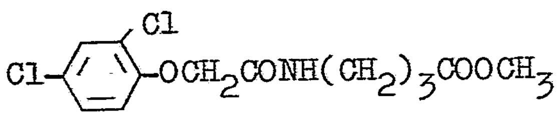

(44) 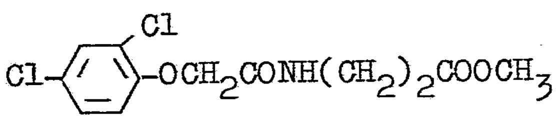

(45) 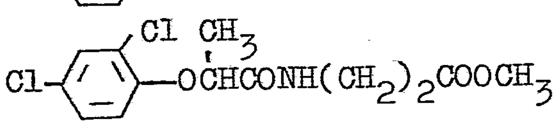

(46) 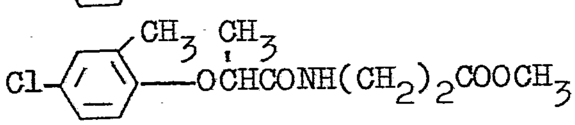

(47) 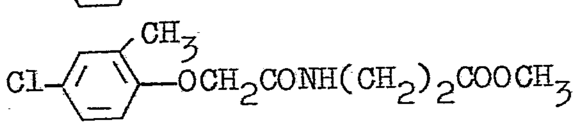

(48) 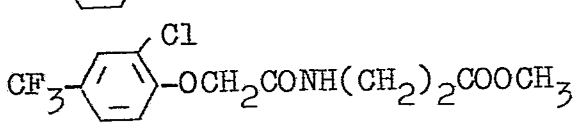

(49) 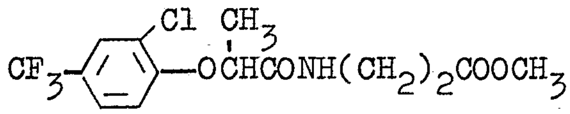

(50) 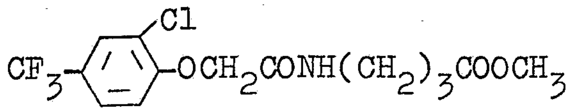

(51) 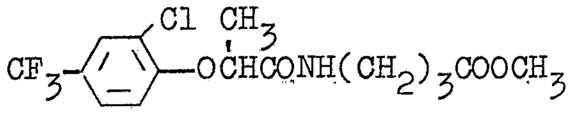

(52) 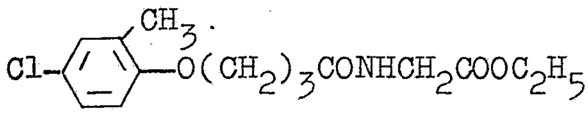

(53) 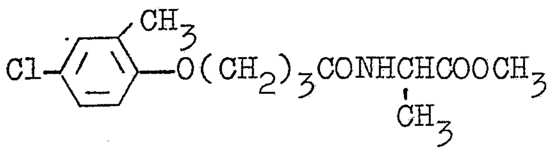

(54) 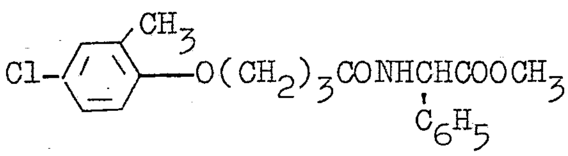

(55) 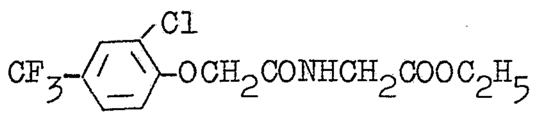

(56) 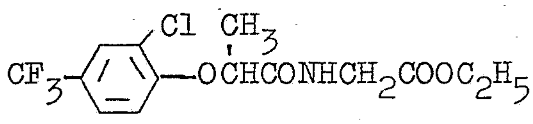

(57) 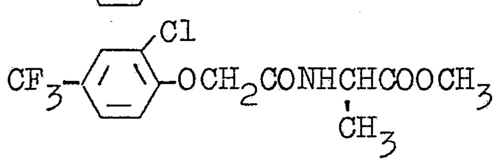

(58) 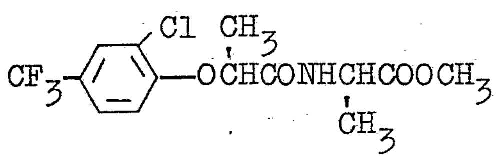

(59) 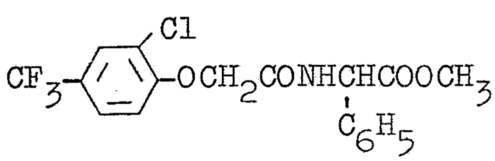

(60) 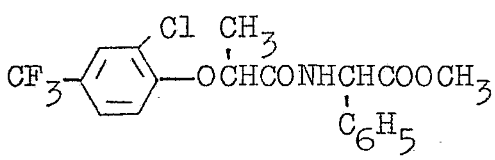

(61) 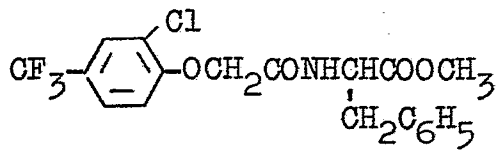

(62) 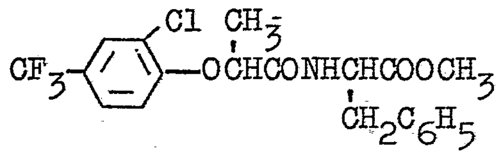

(63) 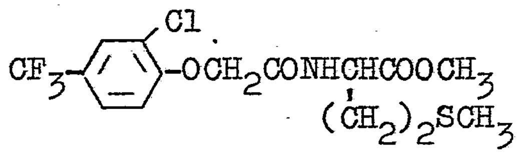

(64) 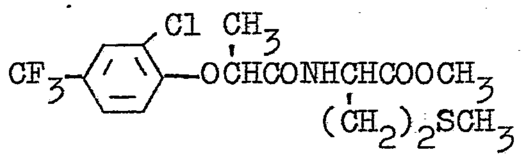

(65) 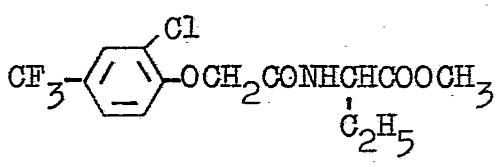

(66) 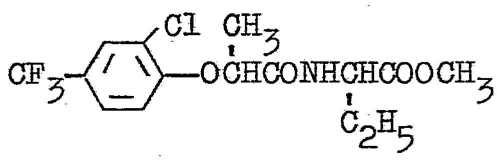

(67) 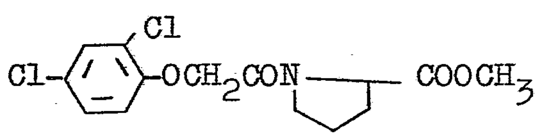

(68) 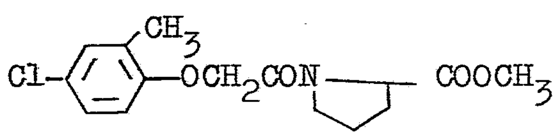

(69) 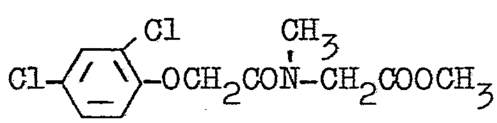

(70) 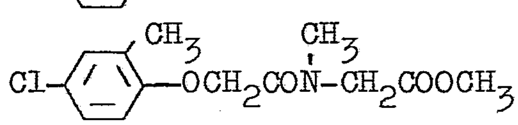

(71) 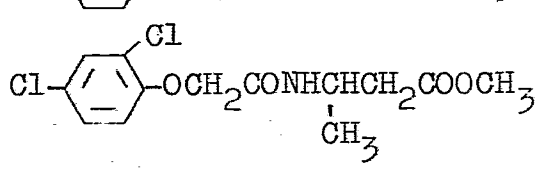

(72) 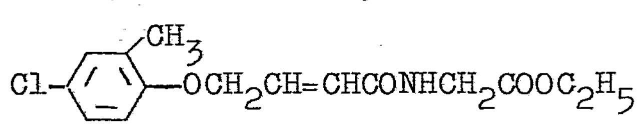

(73) 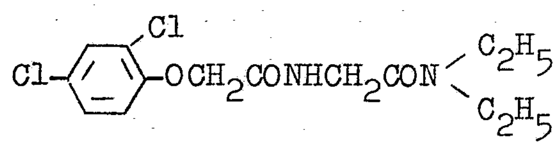

(74) 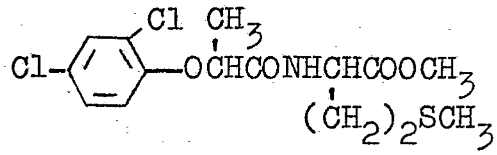

(75) 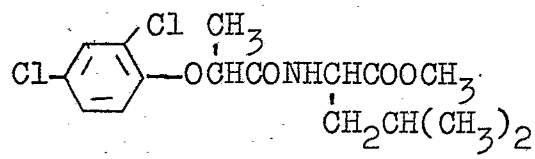

(76) 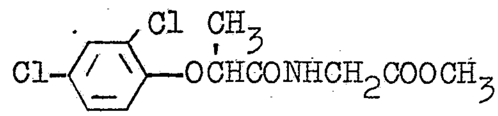

(77) 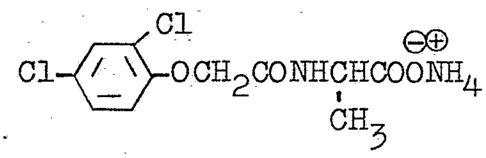

(78) 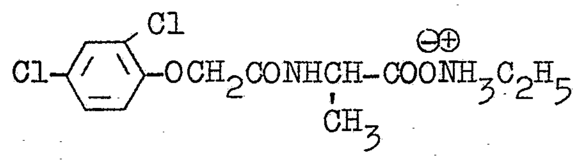

(79) 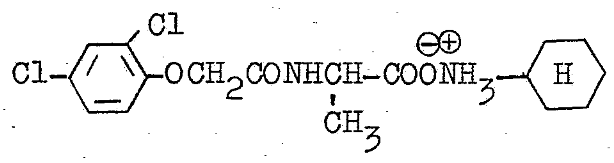

(80) 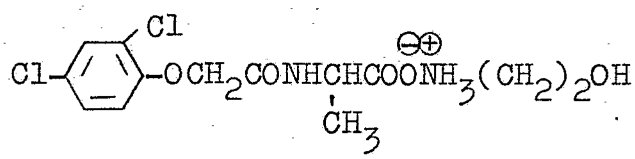

(81) 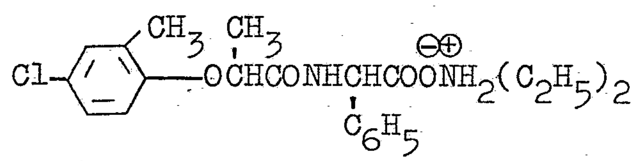

(82) 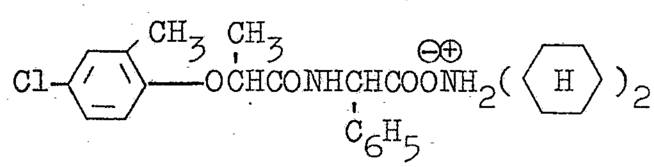

(83) 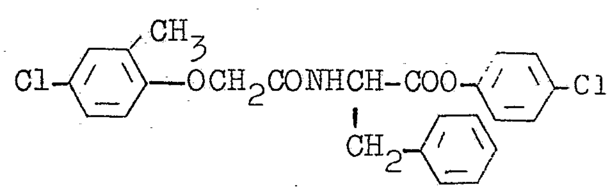

The compounds of general formula (I) in accordance with this invention can be produced, for example, by the following processes.

Process 1

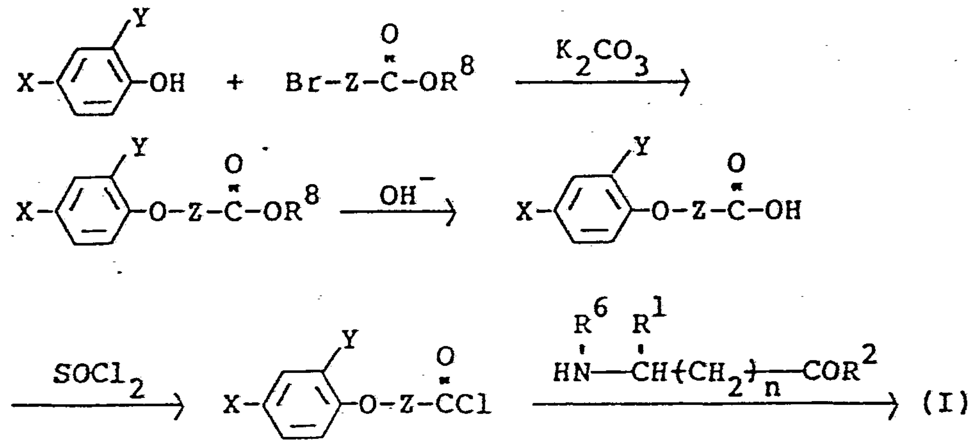


Process 2

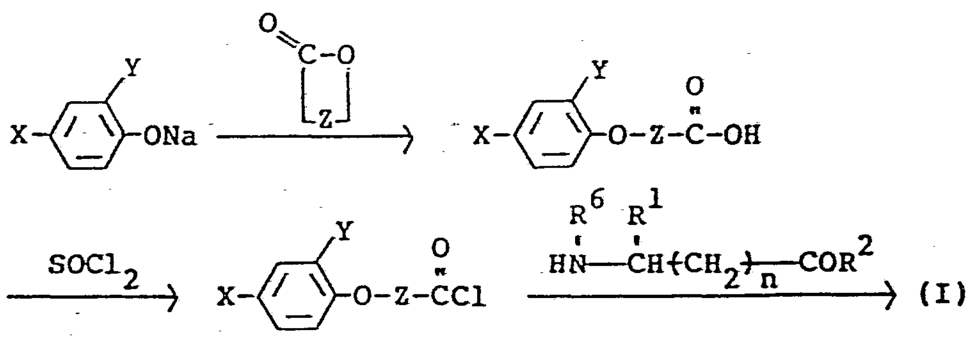


Process 3

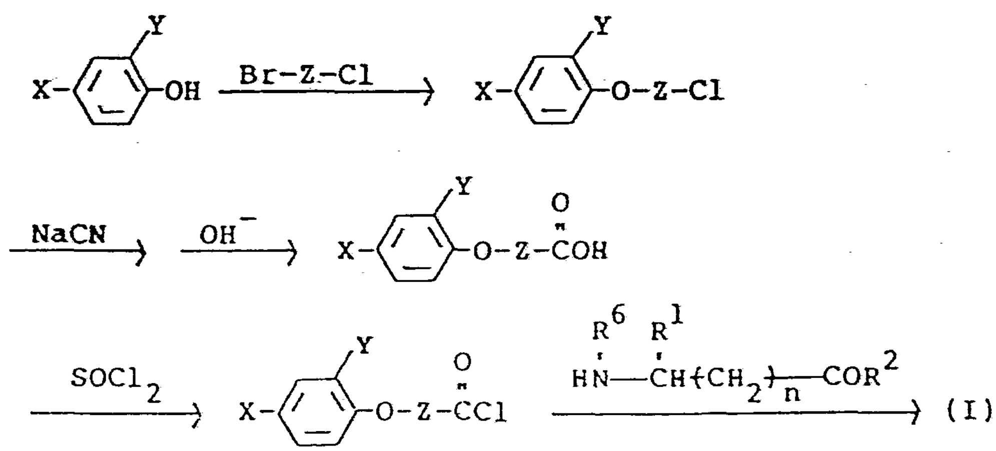

In process 1, $R^8$ represents a lower alkyl group. A compound of geneal formula (1) in which $R^2$ is $OR^3$ and $R^3$ is an ammonium cation may be obtained, for example, by reacting the compound corresponding to general formula (1) in which $R^2$ is OH with ammonia or an amine by a method known *per se* to form a salt. An amide compound of general formula (1) in which $R^2$ is $-N{<}^{R^4}_{R^5}$ may be obtained by amidating the compound corresponding to formula (1) in which $R^2$ is OH, with an amine of the formula $HN{<}^{R^4}_{R^5}$ in accordance with a general synthesizing method.

The compounds of general formula (1) affect the metabolism of plants. They inhibit the growth of certain plant species, regulate the growth of certain plant species, dwarf certain plant species, or wither certain plant species.

Among the above amino acids, glycine, alanine, beta-alanine, valine, isoleucine, leucine, methionine, phenylalanine and phenylglycine are especially preferred for the objects of this invention.

Among the compounds of formula (1), those of the following formula (1)-a

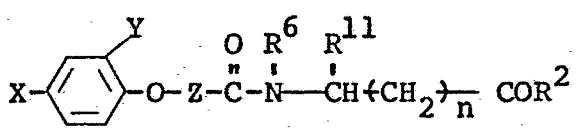 ... (1)-a

wherein X, Y, Z, $R^6$, $R^2$ and n are as defined in formula (1), and $R^{11}$ is a hydrogen atom or a saturated aliphatic hydrocarbon group having not more than 4 carbon atoms,

are preferred active compounds which are substantially non-toxic to corn, for example, and used for eradicating weeds growing in an area where corn is cultivated.

A herbicide comprising as an active ingredient a compound of the following general formula (1)-c which is within general formula (1)

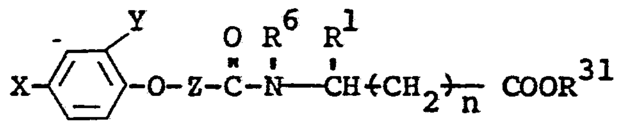 ...(1)-c

wherein X, Y, Z, $R^6$, $R^1$ and n are as defined in formula (1), and $R^{31}$ represents an aliphatic hydrocarbon group having not more than 15 carbon atoms which may be interrupted by an oxygen atom and may have a substituent,

is preferred and a herbicide comprising as an active ingredient a compound of the following general formula (1)-d which is within general formula (1)

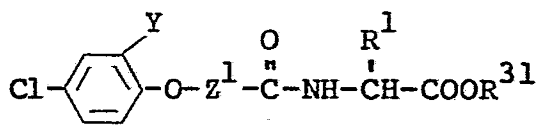 ... (1)-d

wherein Y and $R^1$ are as defined in formula (1), $R^{31}$ is as defined in formula (1)-c, and $Z^1$ is $-CH_2-$ or $-\overset{CH_3}{\overset{|}{C}}H-$,

is more preferred, because they show excellent herbicidal activity.

A herbicide comprising as an active ingredient a compound of the following formula (1)-e

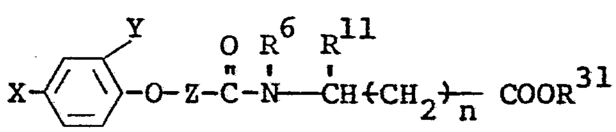 ...(1)-e

wherein X, Y, Z, $R^6$, $R^{11}$ and $R^{31}$ are as defined above,

particularly, a compound of the following formula (1)-f

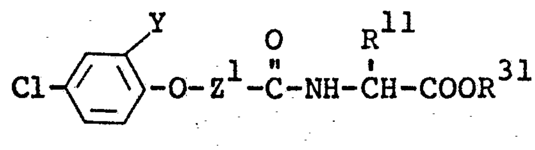 ... (1)-f

wherein X, $Z^1$, $R^{11}$ and $R^{31}$ are as defined above, is very advantageously used to combat weeds in corn fields.

Among the compounds of formula (1), those of the following formula (1)-b

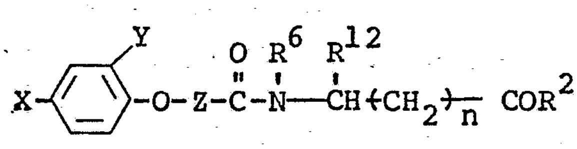 ... (1)-b

wherein X, Y, Z, $R^6$, $R^2$ and n are as defined in formula (1), and $R^{12}$ is phenyl, benzyl or hydroxyphenyl,

are preferred active compounds which are substantially non-toxic to rice, for example, and are used for eradicating weeds in an area where rice is cultivated.

A herbicide comprising as an active ingredient a compound of the following general formula (1)-g which is within general formula (1)

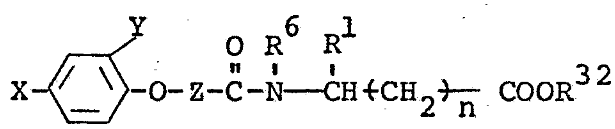 ...(1)-g

wherein X, Y, Z, $R^6$, $R^1$ and n are as defined above and $R^{32}$ represents an aliphatic hydrocarbon group having not more than 15 carbon atoms which may be interrupted by an oxygen atom and may have a substituent, or a phenyl group which may have a substituent,

is preferred, and a herbicide comprising as an active ingredient a compound of the following general formula (1)-h

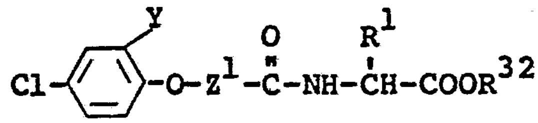 ... (1)-h

wherein Y, $Z^1$, $R^1$ and $R^{32}$ are as defined above, is more preferred, because they shows excellent herbicidal activity.

A herbicide comprising as an active ingredient a compound represented by the following formula (1)-i

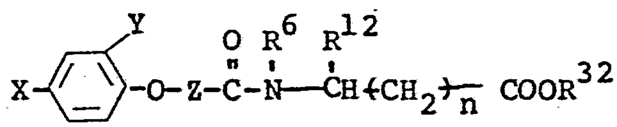 ... (1)-i

wherein X, Y, Z, $R^6$, $R^{12}$, $R^{32}$ and n are as defined above,

particularly a compound represented by the following formula (1)-j

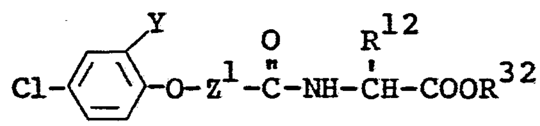 ... (1)-j

wherein Y, $Z^1$, $R^{12}$ and $R^{32}$ are as defined above,

is very advantageously used to combat weeds in corn fields.

The compounds of formula (1) may be applied to plant seeds, or to plants in various growing stages through stalks and leaves or roots. Specifically, the compounds of this invention, either as such or in the form of a composition, can be applied in a herbicidally sufficient amount to the seeds, stalks, leaves or roots of plants to be controlled, or their habitat, or the locus where the growth of such plants is anticipated.

Preferably, the "herbicidally sufficient amount" is, for example, an amount which is sufficient for inhibiting the growth of, or eradicating, plants to be controlled.

The compounds of this invention may be applied to plants in an amount of 10g to 20kg per hectare, preferably 50g to 10kg per hectare, especially preferably 100g to 2 kg per hectare.

When it is desired to inhibit the growth of, or eradicate, noxious plants, the compounds of the invention, either as such or in the form of a composition, may be applied in herbicidally sufficient amounts to the locus where plants to be controlled or their seeds and a useful plant or its seeds are likely to grow together, whereby the plants to be controlled or their seeds can be selectively inhibited in growth or eradicated. For example, the compound of this invention represented by formula (1) can be applied to the locus in which rice or corn as the useful plant is growing or which is to be used for cultivating the above useful plant, in an amount sufficient to control undesired plants or weeds which are growing, or are liketly to grow, in the above locus.

The plants to be controlled are generally noxious plants. The noxious plants may be defined as plants which come into an environment created by man, such as a lowland or upland farm, from the surrounding nature and grow there and which, man recognizes, are not useful, or do harm, in that environment.

These noxious plants are generally called weeds. The compounds of this invention are applicable to various weeds exemplified below.

| Family | Species |
|---|---|
| Polygonaceae | Polygonum hydropiper L. |
| Chenopodiaceae | Chenopodium album L. |
| Ditto | Chenopodium album L. var. centrorubrum Makino |
| Ditto | Chenopodium ficifolium Smith |
| Portulacaceae | Portulaca oleracea L. |
| Amaranthaceae | Amaranthus lividus L. |
| Ditto | Amaranthus retroflexus L. |

| | |
|---|---|
| Compositae | Erigeron annuus L. |
| Plantaginaceae | Plantago asiatica L. |
| Gramineae | Digitaria adscendens Henr. |
| Ditto | Echinochloa crus-galli P. Beauv. |
| Cyperaceae | Cyperus rotundus L. |
| Ditto | Cyperus serotinus Rottb. |
| Ditto | Scirpus hotarui Ohwi |
| Ditto | Eleocharis acicularis Roem. et Schult. var. longiseta Sven. |
| Alismataceae | Sagittaria pygmaea Miq. |
| Pontederiaceae | Monochoria vaginalis Presl. |

The useful plants herein are, for example, plants which produce cereals, or lawn. Since the compounds of this invention exert little or no adverse effects on the growth of rice and corn, they are very suitable for application to farms where such plants are cultivated. By applying the compounds of this invention to an area where lawn is growing, the germination and growth of weeds can be inhibited.

In some cases, it is desirable to apply the compounds of this invention before noxious plants grow to a large height, especially before the height of the noxious plants exceeds that of a useful plant or when the noxious plants are only slightly taller than the useful plant.

The herbicide of this invention exhibits a very good effect against broad-leaved annual and perennial weeds. It also shows herbicidal activity on narrow-leaved plants if their height is small. In some cases, it has sufficient herbicidal activity on narrow-leaved perennial weeds such as Cyperus rotundus L. The herbicide of the invention shows particularly superior herbicidal activity on weeds of the families Compositae (for example, Erigeron sp.), Chenopodiaceae, Polygonaceae, and Portulacaceae (for example, Portulaca oleracea L.).

When it is desired to inhibit the growth of, or eradicate, only noxious plants without substantially harming rice as a useful plant in the locus where rice or its seeds and the noxious plants or their seeds grow together or are likely to grow together, the use of the compounds of formula (1)-b is preferred. Especially preferred compounds of formula (1)-b are those in which

X: Cl

Y: Cl or $-CH_3$

Z: $-CH_2-$, $-CH(CH_3)-$, or $-(CH_2)_3-$

$R^1$: phenyl or benzyl

$R^6$: H

n: 0 or 1

$R^2$: $-OCH_3$, $-OC_2H_5$, $-OC_3H_7$ or -Ophenyl.

If the useful plant is corn, the use of the compounds of formula (1)-a is preferred in the above weed controlling method. Especially preferred compounds of formula (1)-a are those in which

X: Cl

Y: Cl or $-CH_3$

Z: $-CH_2$, $-CH(CH_3)-$ or $-(CH_2)_3-$

$R^1$: H or $-CH_3$

$R^6$: H

n: 0 or 1

$R^2$: $-OCH_3$, $-OC_2H_5$, $-OC_3H_7$, $-OC_4H_9$

The compounds in accordance with this invention may be used as ordinary formulations such as a solution, emulsifiable concentrate, suspension, dust, paste or granules by including carriers and/or surfactants.

Examples of such carriers and surfactants are solid carriers such as talc, bentonite, clay, kaolin, diatomaceous earth, white carbon, vermiculite, slaked lime, ammonium sulfate and urea; liquid carriers such as water, alcohols, dioxane, acetone, xylene, cyclohexane, methylnaphthalene and dimethylformamide; surfactants, emulsifiers, or dispersants such as alkylsulfuric acid esters, alkylsulfonic acid salts, ligninsulfonic acid salts, polyoxyethylene glycol ethers, polyoxyethylene alkyl aryl ethers, polyoxyethylene sorbitan monoalkylates and dinaphthylmethanedisulfonic acid salts; various auxiliary agents such as carboxymethyl cellulose and gum arabic. Such formulations can be prepared by, for example, mixing the compounds of this invention with the above-exemplified carriers and/or emulsifiers, etc.

The compounds of this invention may exist in a concentration of usually 0.01 to 99% by weight, preferably 0.1 to 96% by weight, in the formulations.

The compounds of this invention, either as such or as a mixutre with other active compounds or in the form of the above formulations, can be applied to plants by usual methods such as spraying, atomizing, scattering or dusting.

The following Examples illustrate the present invention in greater detail.

In these examples, all parts are by weight. The herbicidal activities of the compounds were evaluated on a scale of 6 grades unless otherwise specified. Specifically, a rating of 0 means that after the application of the active compound, a plant is as healthy as it was before the application, and a rating of 5 means that the entire plant withers and dies after the application of the active compound. Ratings of 1, 2, 3 and 4 are assigned to varying enfeebled conditions of the plant between the above two extremities.

PRODUCTION EXAMPLE

By using the processes shown hereinabove, compounds of formula (1) were prepared. The properties of these compounds are shown in Table 1.

Table 1

| Compound No. | IR $\nu(cm^{-1})$ | NMR solvent | $\delta$(ppm) |
|---|---|---|---|
| 1 | 3400<br>1740<br>1660<br>1540 | $CC\ell_4$ | 1.30(3H),<br>4.23(2H),<br>4.27(2H),<br>4.50(2H),<br>6.80(1H),<br>6.9-7.5(3H) |
| 2 | 3380<br>1740<br>1650<br>1560 | $CDC\ell_3$ | 1.47(3H),<br>3.80(3H),<br>4.50(2H),<br>4.73(1H),<br>6.85(1H),<br>7.1-7.5(3H) |
| 3 | 3400<br>1740<br>1660 | $CDC\ell_3$ | 3.30(2H),<br>3.67(3H),<br>4.43(2H),<br>4.87(1H),<br>6.67(1H),<br>7.0-7.4(8H) |
| 4 | 3250<br>1740<br>1660 | $CDC\ell_3$ | 3.73(3H),<br>4.53(2H),<br>5.63(1H),<br>6.70-7.40(8H),<br>7.67-7.92(1H) |
| 5 | 3400<br>1740<br>1660<br>1540 | $CDC\ell_3$ | 3.80(3H),<br>4.12(2H),<br>4.53(2H),<br>6.83(1H),<br>7.0-7.4(4H) |

- to be continued -

Table 1 (continued)

| Compound No. | IR $\nu(cm^{-1})$ | NMR solvent | NMR $\delta$(ppm) |
|---|---|---|---|
| 6 | 3300<br>1730<br>1660<br>1580 | $CC\ell_4$ | 0.9(3H),<br>1.6-2.0(2H),<br>3.7(3H),<br>4.5(2H),<br>4.5-4.7(1H),<br>6.65-7.4(4H) |
| 7 | 3400<br>1740<br>1670<br>1520 | $CDC\ell_3$ | 0.9-1.0(6H),<br>1.55-1.7(3H),<br>4.5(2H),<br>3.8(3H),<br>4.5-4.7(1H),<br>6.8-7.5(4H) |
| 8 | 3400<br>1740<br>1670 | $CC\ell_4$ | 2.03(3H),<br>2.03-2.60(4H),<br>3.73(3H),<br>4.47(2H),<br>4.50-4.80(1H),<br>6.73-7.37(4H) |
| 9 | 3400<br>1740<br>1680 | $CDC\ell_3$ | 3.77(3H),<br>4.50-4.57(2H),<br>4.67(2H),<br>4.77-5.03(1H),<br>6.63-7.67(5H) |
| 10 | 3400<br>1740<br>1680 | $CC\ell_4$ | 0.95(6H),<br>1.28(3H),<br>1.8-2.4(1H),<br>4.17(2H),<br>4.47(2H),<br>4.50(1H),<br>6.8-7.4(4H) |

Table 1 (continued)

| Compound No. | IR ν($cm^{-1}$) | NMR solvent | NMR δ(ppm) |
|---|---|---|---|
| 11 | 3400<br>1740<br>1680 | $CCl_4$ | 1.25(3H),<br>2.83(2H),<br>3.67(2H),<br>4.18(2H),<br>4.35(2H),<br>4.75(1H),<br>6.80(1H),<br>7.0-7.4(8H) |
| 12 | 3300<br>1740<br>1650<br>1580 | $CDCl_3$ | 1.7(3H),<br>3.7(3H),<br>4.5-4.9(1H),<br>5.5(1H),<br>6.6-7.3(8H),<br>7.4-8.0(1H) |
| 13 | 3300<br>1740<br>1660<br>1540 | $CDCl_3$ | 1.0(3H),<br>1.3(3H),<br>3.8-4.1(4H),<br>4.2-4.6(1H),<br>6.5-7.1(4H) |
| 14 | 3300<br>1740<br>1650<br>1560 | $CDCl_3$ | 1.3-1.7(6H),<br>3.7-3.75(3H),<br>4.45-4.85(2H),<br>6.7-7.5(4H) |
| 15 | 3300<br>1740<br>1660 | $CDCl_3$ | 1.57(3H),<br>3.15(2H),<br>3.73(3H),<br>4.4-5.1(2H),<br>6.60(1H),<br>6.8-7.4(8H) |

- to be continued -

Table 1 (continued)

| Compound No. | IR ν(cm-1) | solvent | NMR δ (ppm) | |
|---|---|---|---|---|
| 16 | 3250<br>1745<br>1660<br>1580 | $CDCl_3$ | 0.95(3H),<br>1.1-2.0(4H),<br>4.20(2H),<br>4.20(2H), | 4.56(2H),<br>6.83(1H),<br>7.1-7.5(3H) |
| 17 | 3230<br>3080<br>1725<br>1655<br>1560 | $CDCl_3$ | 0.95(3H),<br>1.1-2.0(4H),<br>1.50(3H),<br>4.20(2H), | 4.53(2H),<br>4.73(1H),<br>6.83(1H),<br>7.1-7.5(3H) |
| 18 | 3250<br>3100<br>1735<br>1660<br>1560 | $CDCl_3$ | 0.93(6H)<br>1.4-2.0(3H),<br>1.47(3H),<br>4.20(2H), | 4.5(2H),<br>4.72(1H)<br>6.83(1H),<br>7.1-7.5(3H) |
| 19 | 3400<br>1735<br>1670<br>1560 | $CDCl_3$ | 1.83(3H),<br>1.0-1.9(8H),<br>4.13(2H),<br>4.20(2H), | 4.55(2H),<br>6.83(1H),<br>7.1-7.5(3H) |
| 20 | 3400<br>1750<br>1640 | $CDCl_3$ | 1.6(3H),<br>2.25(3H),<br>3.7(3H), | 4.5-4.9(1H),<br>5.55(1H),<br>6.5-7.7(9H) |
| 21 | 3400<br>1740<br>1650<br>1550 | $CDCl_3$ | 1.25(3H),<br>1.55(2H),<br>2.3(3H), | 4.0-4.4(4H),<br>4.45-4.85(1H),<br>6.6-7.55(4H) |

- to be continued -

Table 1 (continued)

| Compound No. | IR ν(cm$^{-1}$) | NMR solvent | δ(ppm) |
|---|---|---|---|
| 22 | 3400<br>1740<br>1660<br>1560 | $CDCl_3$ | 1.3-1.6(6H),<br>2.3(3H),<br>3.7-3.75(3H),<br>4.45-4.85(2H),<br>6.6-7.7(4H) |
| 23 | 3400<br>1740<br>1650<br>1550 | $CCl_4$ | 1.30(3H),<br>2.30(3H),<br>4.05(2H),<br>4.20(2H),<br>4.43(2H),<br>6.63(1H),<br>6.9-7.3(3H) |
| 24 | 3400<br>1740<br>1660<br>1560 | $CDCl_3$ | 1.47(3H),<br>2.30(3H),<br>3.77(3H),<br>4.47(2H),<br>4.67(1H),<br>6.67(1H),<br>7.0-7.3(3H) |
| 25 | 3300<br>1730<br>1660 | $CDCl_3$ | 2.10(3H),<br>3.15(2H),<br>3.70(3H),<br>4.40(2H),<br>4.93(1H),<br>6.57(1H),<br>6.9-7.3(8H) |

- to be continued -

Table 1 (continued)

| Compound No. | IR ν(cm-1) | solvent | NMR δ(ppm) |
|---|---|---|---|
| 26 | 3350<br>1730<br>1660<br>1530 | $CDCl_3$ | 2.30(3H), 3.73(3H), 4.47(2H), 5.63(1H), 6.53-7.82(8H) |
| 27 | 3400<br>1740<br>1670 | $CCl_4$ | 2.00(3H), 2.30(3H), 2.03-2.57(4H), 3.73(3H), 4.40(2H), 4.57-4.80(1H), 6.60-7.40(4H) |
| 28 | 3300<br>1730<br>1660 | $CDCl_3$ | 1.57(3H), 2.17(3H), 3.18(2H), 3.73(3H), 4.60(1H), 4.90(1H), 6.4-7.4(8H) |
| 29 | 3350<br>1735<br>1655<br>1560 | $CDCl_3$ | 0.95(3H), 1.1-2.0(4H), 1.50(3H), 2.33(3H), 4.20(2H) 4.50(2H), 4.75(1H), 6.70(1H), 7.0-7.4(3H) |
| 30 | 3380<br>1740<br>1650<br>1550 | $CDCl_3$ | 2.303H), 3.80(3H), 4.12(2H), 4.53(2H), 6.8-7.4(4H) |

- to be continued -

Table 1 (continued)

| Compound No. | IR ν($cm^{-1}$) | NMR solvent | δ(ppm) |
|---|---|---|---|
| 31 | 3400<br>1735<br>1680<br>1620 | $CCl_4$ | 1.43(3H), 4.45(2H), 4.56(1H), 4.63(2H), 5.06-5.23(1H), 5.23-5.50(1H), 5.60-6.20(1H), 6.85(1H), 7.06-7.46 (3H) |
| 32 | 3400<br>1740<br>1680 | $CCl_4$ | 0.96(3H), 1.45(3H), 1.60(2H), 4.06(2H), 4.42(2H), 4.55(1H), 6.83(1H), 7.0-7.4(3H) |
| 33 | 3400<br>1740<br>1680 | $CCl_4$ | 0.90(3H), 1.43(3H), 1.0-2.0(6H), 4.10(2H), 4.43(2H), 4.5-5.0(1H), 6.80(1H), 7.0-7.4 (3H) |
| 34 | 3400<br>1740<br>1675 | $CCl_4$ | 0.85(3H), 1.0-1.9 (16H), 1.43(3H), 4.10(2H), 4.43(2H), 4.3-4.7(1H), 6.80 (1H), 7.0-7.4(3H) |
| 35 | 3400<br>1735<br>1675 | $CCl_4$ | 1.43(3H), 1.1-2.1 (10H), 4.43(2H), 4.3-4.6(1H), 4.5-5.0(1H), 6.85(1H), 7.1-7.5 (3H) |

- to be continued -

Table 1 (continued)

| Compound No. | IR ν(cm$^{-1}$) | NMR solvent | NMR δ(ppm) |
|---|---|---|---|
| 36 | 3400<br>1740<br>1680 | $CCl_4$ | 1.45(3H), 3.25(3H), 3.50(4H), 4.45(2H), 4.3-4.8(1H), 6.85(1H), 7.1-7.5 (1H) |
| 37 | 3360<br>3280<br>1735<br>1660 | $CCl_4$ | 1.05(3H), 1.22(3H), 1.50(3H), 2.30(3H), 4.55(1H), 4.95(1H), 5.36(1H), 6.48(1H), 6.7-7.5(8H) |
| 38 | 3360<br>3280<br>1735<br>1660 | $CDCl_3$ | 0.80(3H), 0.9-1.6 (6H), 1.60(3H), 2.30(3H), 4.10(2H), 4.65(1H), 5.50(1H), 6.4-7.7(9H) |
| 39 | 3360<br>3280<br>1780<br>1660 | $CDCl_3$ | 0.82(3H), 1.0-1.5 (12H), 1.60(3H), 2.30(3H), 4.10(2H), 4.65(1H), 5.50(1H), 6.4-7.6(9H) |
| 40 | 3360<br>3280<br>1730<br>1660 | $CDCl_3$ | 0.95(3H), 1.1-1.5 (26H), 1.60(3H), 2.30(3H), 4.10(2H), 4.70(1H), 5.60(1H), 6.5-7.7(9H) |

- to be continued -

Table 1 (continued)

| Compound No. | IR ν($cm^{-1}$) | NMR solvent | NMR δ(ppm) |
|---|---|---|---|
| 41 | 3270<br>1655 | $CDCl_3$ | 1.40(3H), 1.1-2.1 (6H), 3.3-3.9(4H), 4.50(2H), 4.97(1H), 6.80(1H), 7.0-7.5 (2H), 7.8-8.1(1H) |
| 42 | 3300<br>3270<br>1660 | $CDCl_3$ | 1.25(6H), 3.8-4.5(3H), 4.45(2H), 6.80(1H), 6.9-7.7(4H) |
| 43 | 3250<br>1740<br>1650<br>1570 | $CDCl_3$ | 1.8-2.1(2H), 2.2-2.5(2H), 3.2-3.5(2H), 3.65(3H), 4.5(2H), 6.7-7.4(4H) |
| 44 | 3400<br>1720<br>1680<br>1520 | $CDCl_3$ | 2.5-2.7(2H), 3.5-3.75(2H), 3.7(3H), 4.5(2H), 6.7-7.4(4H) |
| 45 | 3250<br>1730<br>1660<br>1560 | $CDCl_3$ | 1.6(3H), 2.4-2.6(2H), 3.4-3.6(2H), 3.6(3H), 4.5-4.85(1H), 6.7-7.4(3H) |
| 46 | 3250<br>1730<br>1660<br>1560 | $CDCl_3$ | 1.5(3H), 2.2(3H), 2.3-2.5(2H), 3.3-3.4(2H), 4.3-4.7(1H), 6.5-7.4(3H) |

- to be continued -

Table 1 (continued)

| Compound No. | IR ν($cm^{-1}$) | NMR solvent | δ(ppm) |
|---|---|---|---|
| 47 | 3400<br>1730<br>1680<br>1520 | $CDCl_3$ | 2.2(3H),<br>2.5-2.7(2H),<br>3.4-3.6(2H),<br>3.7(3H), 4.45(2H),<br>6.65-7.3(4H) |
| 48 | 3400<br>1730<br>1660<br>1600<br>1530 | $CDCl_3$ | 2.5-2.7(2H),<br>3.7(3H),<br>3.5-3.75(2H),<br>4.5(2H),<br>6.9-7.7(4H) |
| 49 | 3250<br>1730<br>1650<br>1610<br>1560 | $CDCl_3$ | 1.6(3H),<br>2.4-2.6(2H),<br>3.4-3.6(2H),<br>3.6(3H),<br>4.6-4.95(1H),<br>6.9-7.7(4H) |
| 50 | 3260<br>1720<br>1660<br>1560 | $CDCl_3$ | 1.7-2.0(2H),<br>2.2-2.5(2H),<br>3.2-3.5(2H),<br>3.7(3H), 4.55(2H),<br>6.8-7.7(4H) |
| 51 | 3250<br>1730<br>1650<br>1600<br>1540 | $CDCl_3$ | 1.6(3H),<br>1.7-2.1(2H),<br>2.2-2.5(2H),<br>3.2-3.4(2H),<br>3.65(3H),<br>4.6-4.95(1H),<br>6.9-7.7(4H) |

- to be continued -

Table 1 (continued)

| Compound No. | IR $\nu(cm^{-1})$ | NMR solvent | $\delta$(ppm) |
|---|---|---|---|
| 52 | 3400<br>1740<br>1650<br>1560 | $CC\ell_4$ | 1.27(3H), 2.17(3H),<br>1.9-2.7(4H),<br>3.90(2H), 3.93(2H),<br>4.13(2H),<br>6.2-6.6(1H),<br>6.60(1H),<br>6.8-7.1(2H) |
| 53 | 3390<br>1740<br>1650<br>1560 | $CDC\ell_3$ | 1.41(3H), 2.20(3H),<br>1.9-2.7(4H),<br>3.73(3H), 3.97(2H),<br>4.60(1H),<br>5.9-6.3(1H),<br>6.70(1H),<br>6.9-7.4(2H) |
| 54 | 3400<br>1740<br>1650<br>1560 | $CDC\ell_3$ | 2.60(3H),<br>1.8-2.6(4H),<br>3.73(3H), 3.93(2H),<br>5.60(1H),<br>6.5-6.8(2H),<br>6.9-7.3(2H),<br>7.33(5H) |
| 55 | 3400<br>1740<br>1660<br>1610<br>1520 | $CDC\ell_3$ | 1.3(3H),<br>4.05-4.4(4H),<br>4.6(2H),<br>6.7-7.7(4H) |
| 56 | 3300<br>1740<br>1660 | $CDC\ell_3$ | 1.3(3H), 1.65(3H),<br>4.0-4.55(4H),<br>4.7-5.0(1H),<br>6.9-7.7(4H) |

- to be continued -

Table 1 (continued)

| Compound No. | IR ν($cm^{-1}$) | NMR solvent | NMR δ(ppm) |
|---|---|---|---|
| 57 | 3400<br>1750<br>1680<br>1600<br>1530 | $CDCl_3$ | 1.5(3H), 3.8(3H),<br>4.55(2H),<br>4.5-4.9(1H),<br>6.9-7.7(4H) |
| 58 | 3250<br>1750<br>1650<br>1550 | $CDCl_3$ | 1.3-1.7(6H),<br>3.7-3.75(3H),<br>4.5-4.9(2H),<br>6.9-7.7(4H) |
| 59 | 3400<br>1740<br>1660<br>1600<br>1500 | $CDCl_3$ | 3.7(3H),<br>4.6(2H),<br>5.6(1H),<br>6.8-7.9(9H) |
| 60 | 3400<br>1740<br>1640<br>1600 | $CCl_4$ | 1.7(3H), 3.7(3H),<br>4.6-4.9(1H),<br>5.4(1H),<br>6.7-7.7(9H) |
| 61 | 3250<br>1740<br>1690<br>1610<br>1560 | $CDCl_3$ | 3.1(2H),<br>3.7(3H),<br>4.5(2H),<br>4.7-5.1(1H),<br>6.8-7.7(9H) |
| 62 | 3300<br>1750<br>1660<br>1600<br>1550 | $CDCl_3$ | 1.6(3H),<br>3.2(2H),<br>3.7(3H),<br>4.5-5.0(2H),<br>6.7-7.6(9H) |

- to be continued -

Table 1 (continued)

| Compound No. | IR ν($cm^{-1}$) | NMR solvent | NMR δ(ppm) |
|---|---|---|---|
| 63 | 3350<br>1740<br>1680<br>1520 | $CDCl_3$ | 2.05(3H),<br>2.1-2.7(4H),<br>3.8(3H), 4.6(2H),<br>4.8-5.0(1H),<br>6.9-7.7(4H) |
| 64 | 3250<br>1740<br>1660<br>1600<br>1540 | $CDCl_3$ | 1.6-1.8(3H),<br>2.0-2.1(3H),<br>1.9-2.7(4H),<br>3.8(3H),<br>4.6-5.0(2H),<br>6.8-7.7(4H) |
| 65 | 3400<br>1740<br>1680<br>1610 | $CCl_4$ | 0.9(3H),<br>1.6-2.1(2H),<br>3.7(3H), 4.4(2H),<br>4.4-4.7(1H),<br>6.8-7.7(4H) |
| 66 | 3250<br>1740<br>1660<br>1610<br>1540 | $CDCl_3$ | 0.8(3H), 1.7(3H),<br>1.5-2.0(2H),<br>3.75(3H),<br>4.4-5.0(2H),<br>6.9-7.7(4H) |
| 67 | 1735<br>1665<br>1655 | $CDCl_3$ | 1.7-2.4(4H),<br>3.70(3H),<br>3.6-3.8(2H),<br>4.65(1H), 4.73(2H),<br>6.87(1H),<br>7.0-7.4(2H) |

- to be continued -

Table 1 (continued)

| Compound No. | IR ν($cm^{-1}$) | NMR solvent | NMR δ(ppm) |
|---|---|---|---|
| 68 | 1740<br>1640<br>1630 | $CDCl_3$ | 1.7-2.4(4H), 2.25(3H), 3.70(3H), 3.5-3.8(2H), 4.55(1H), 4.67(2H), 6.70(1H), 6.9-7.3(2H) |
| 69 | 1740<br>1675<br>1660 | $CDCl_3$ | 3.20(3H), 3.75(3H), 4.13(2H), 4.85(2H), 6.8-7.3(3H) |
| 70 | 1740<br>1655 | $CDCl_3$ | 2.30(3H), 3.20(3H), 3.77(3H), 4.17(2H), 4.78(2H), 6.6-7.3(3H) |
| 71 | 3300<br>1740<br>1650<br>1560 | $CDCl_3$ | 1.27(3H), 2.57(2H), 3.70(3H), 4.47(2H), 4.20-4.67(1H), 6.70-7.43(4H) |
| 72 | 3300<br>1740<br>1660<br>1620 | $CDCl_3$ | 1.67(3H), 2.23(3H), 4.10(2H), 4.20(2H), 4.65(2H), 6.15(1H), 6.5-7.3(5H) |
| 73 | 3280<br>1670<br>1650<br>1560 | $CD_3OD$ | 1.30(6H), 3.07(4H), 3.83(2H), 4.60(2H), 7.0-7.4(4H) |

- to be continued -

Table 1 (continued)

| Compound No. | IR ν (cm-1) | solvent | NMR δ (ppm) |
|---|---|---|---|
| 74 | 3250<br>1740<br>1660 | $CDCl_3$ | 1.63(2H),<br>2.00-2.10(3H),<br>2.10-2.70(4H),<br>3.70-3.76(3H),<br>4.50-4.86(2H),<br>6.76-7.40(4H) |
| 75 | 3280<br>1740<br>1660 | $CDCl_3$ | 0.83-1.00(6H),<br>1.50-1.70(6H),<br>3.67-3.73(3H),<br>4.50-4.90(2H),<br>6.76-7.40(4H) |
| 77 | 3400<br>2140<br>1660<br>1580 | $C_2O$/DMSO-$d_6$(6/4) | 1.36(3H),<br>4.15(1H),<br>4.60(2H),<br>4.62(5H),<br>6.9-7.5(3H) |
| 78 | 3400<br>2100<br>1660<br>1580 | $D_2O$/DMSO-$d_6$(6/4) | 1.3(3H)<br>1.40(3H)<br>3.05(2H)<br>4.22((1H)<br>4.60(2H)<br>4.62(4H)<br>6.9-7.5(3H) |
| 79 | 3380<br>2160<br>1665<br>1600 | DMSO-$d_6$ | 1.25(3H)<br>0.9-2.1(10H)<br>2.5-3.1(1H)<br>3.95(1H)<br>4.60(2H)<br>7.0-7.6(3H)<br>7.6-8.2(4H) |

- to be continued -

Table 1 (continued)

| Compound No. | IR ν (cm-1) | solvent | NMR δ (ppm) |
|---|---|---|---|
| 80 | 3400<br>2100<br>1660<br>1580 | DMSO-$d_6$ | 1.28(3H),<br>2.85(2H),<br>3.60(2H),<br>4.00(1H).<br>4.60(2H),<br>7.0-7.7(7H),<br>7.90(1H) |
| 81 | 3400<br>2500<br>1640 | DMSO-$d_6$ | 1.05(6H),<br>1.45(3H),<br>2.30(3H),<br>2.80(4H),<br>4.85(2H),<br>6.8-7.6(10H),<br>8.20(1H) |
| 82 | 3400<br>2510<br>1670<br>1630 | DMSO-$d_6$ | 1.45(3H),<br>0.8-2.1(20H),<br>2.30(3H),<br>2.5-3.2(2H),<br>4.80(2H),<br>6.7-7.5(8H),<br>7.6-8.5(3H) |
| 83 | 3350<br>3100<br>1755<br>1670<br>1550 | $CDCl_3$ | 2.13(3H)<br>3.30(2H),<br>4.50(2H),<br>5.17(1H),<br>6.63(1H),<br>6.9-7.5(12H) |

FORMULATION EXAMPLE 1

One part of the active compound of the invention was added to 5000 parts of a mixture of acetone and water (1:1 by volume), and further 2.6 parts of a nonionic surfactant (Solpor 2680, a trade name) was added to prepare a solution.

FORMULATION EXAMPLE 2

One part of the active compound of this invention, 8.7 parts of a mixture of equal amounts of talc and bentonite, and 0.3 part of a mixture of equal amounts of Solpor 5060 (trade name) and Solpor 800A (trade name) were well pulverized and mixed to form a wettable powder.

FORMULATION EXAMPLE 3

The active compound of this invention (0.5 part), 8.9 parts of a mixture of equal amounts of talc and bentonite, 0.4 part of Solpor 800A (trade name) and 0.2 part of sodium tripolyphosphate were pulverized and mixed together with a small amount of water. The mixture was granulated by a granulator, and spontaneously dried to prepare 5% granules.

FORMULATION EXAMPLE 4

Thirty parts of compound (16) of the invention was dissolved in a mixed solvent composed of 55 parts of xylene and 5 parts of N,N-dimethylformamide, and 10 parts of an anionic surface-active agent was added to prepare an emulsifiable concentrate.

FORMULATION EXAMPLE 5

Twenty parts of compound (17) of the invention was dissolved in a mixed solvent composed of 30 parts of N,N-dimethylformamide, and 40 parts of alpha-methylnaphthalene, and 10 parts of an anionic surface-active agent was added to prepare an emulsifiable concentrate.

FORMULATION EXAMPLE 6

Twenty-five parts of compound (29) of the invention was dissolved in a mixed solvent composed of 30 parts of N,N-dimethylformamide, and 35 parts of alpha-

methylnaphthalene, and 10 parts of an anionic surface-active agent was added to prepare an emulsifiable concentrate.

EXAMPLES 1-48

Solutions of the active compounds shown in Table 2 were prepared in accordance with Formulation Example 1.

The plants shown in Table 2 and used in the present test were obtained by sowing their seeds in the soil and growing them for 2 to 3 weeks after germination.

Each of the test solutions was applied to the plants in such a dose that the total amount of each active compound was 0.1 $g/m^2$. Thereafter, without applying the solution further, the plants were grown for 3 weeks. Then, the herbicidal activities of the test compounds were evaluated, and evaluated on the scale described hereinabove. The results are shown in Table 2.

The numbers ① to ⑲ designating the test plants mean the following (the same designations are used hereinafter).

① Waterpepper (Polygonum hydropiper L)
② Chenopodium album Linnaeus var. centrorubrum Makino
③ Lambsquarters (Chenopodium album L.)
④ Chenopodium serotinum L.
⑤ Common purslane (Portulaca oleracea L.)
⑥ Livid amaranth (Amaranthus blitum L.)
⑦ Redroot pigweed (Amaranthus retroflexus L.)
⑧ Annual fleabane (Erigeron annuus L.)
⑨ Plantain (Plantago asiatica L.)
⑩ Crabgrass (Digitaria sanguinalis Scopoli)
⑪ Purple nutgrass (Cyperus rotundus L.)
⑫ Monochoria (Monochoria vaginalis Presl.)
⑬ Sagittaria pygmaea Miquel
⑭ Bulrush (Scirpus juncoides Roxburgh var. hotarui Ohwi)
⑮ Cyperus serotinus Rottboell

(16) Spikerush (Eleocharis acicularis Roemer et Schultes)

(17) Baryardgrass (Echinochloa crusqalli (L.) P. Beauv.)

(18) Rice

(19) Corn

The annual fleabane and monochoria are annual broad-leaved plants. Crabgrass and Baryardgrasse are annual narrow-leaved plants. Plantain, Sagittaria pygmaea and bulrush are perennial broad-leaved plants, and purple nut grass, Cyperus serotinus and spikerush are perennial narrow-leaved plants. Waterpepper, Chenopodium album Linnaeus var. centrorubrum Makino, lambsquarters, Chenopodium album L., Livid amaranth, common purslane and Redroot pigweed are annual broad-leaved plants.

EXAMPLES 49-91

Seeds of plants were sown in the soil, and treated in the following manner on the second day after sowing. The growth of the plants was observed for 3 weeks.

A wettable powder containing each of the active compounds of this invention which was prepared in accordance with Formulation Example 2 was suspended in 150 $cc/m^2$ of water so that the total amount of the active compound became 0.1 $g/m^2$. The suspension was uniformly applied to the surface of the soil after the sowing. Thereafter, without further applying the test chemical, the plants were grown. The results are shown in Table 3.

EXAMPLES 92-101

Soil was well mixed with water to create a paddy condition, and seeds of tubers of plants to be evaluated were sown or planted in the soil. Furthermore, rice plants in the 3- to 4-leaf stage were transplanted in the soil and maintained in the irrigated state. The water surface was treated in the following manner, and the growth of the plants was observed for 3 weeks.

A wettable powder containing each of the active

compounds of this invention which was prepared in accordance with Formulation Example 2 was suspended in 500 $cc/m^2$ of water so that the total amount of the compound applied became 0.1 $g/m^2$. The suspension was then applied uniformly to the water surface, and thereafter without further applying the test chemical, the plants were grown. The results are shown in Table 4.

EXAMPLES 102-103

Soil was well mixed with water to provide a paddy condition, and seeds or tubers of plants to be evaluated were sown or planted in the soil. Furthermore, rice plants in the 3- to 4-leaf stage were transplanted in the soil and the soil was maintained in the irrigated state. THe water surface was treated in the following manner, and the growth of the plants was observed for 3 weeks.

Granules containing each of the active compounds of this invention which were prepared in accordance with Formulation Example 3 were applied to the water surface so that the total amount of the active compound became 0.1 $g/m^2$. Thereafter, without further applying the test chemical, the plants were grown. The results are shown in Table 5.

EXAMPLES 104-111

A solution containing each of the active compounds of this invention was prepared as in Examples 1-48, and applied to plants so that the total amount of the active compound applied became 0.05 $g/m^2$. The results are shown in Table 6.

EXAMPLES 112-115

A solution containing each of the active compounds of this invention was prepared as in Examples 1-48, and applied to plants so that the total amount of the active compound applied became 0.025 $g/m^2$. The results are shown in Table 7.

EXAMPLES 116-124

A wettable powder containing each of the active

compounds of this invention was prepared as in Examples 49-91, and applied to plants so that the total amount of the active compound applied became 0.05 g/$m^2$. The results are shown in Table 8.

EXAMPLES 125-126

A wettable powder containing each of the active compounds of this invention was prepared as in Examples 49-91, and applied to plants so that the total amount of the active compound applied became 0.025 g/$m^2$. The results are shown in Table 9.

EXAMPLES 127-132

Granules containing each of the active compounds of this invention were prepared as in Examples 92-101 and applied to plants so that the total amount of the active compound applied became 0.05 g/$m^2$. The results are shown in Table 10.

EXAMPLE 133

The emulsifiable concentrate prepared in Formulation Example 4 (4.6 parts by volume) was diluted with 11.4 parts by volume of water. The emulsion was applied to plants, 2 to 3 weeks after emergence, at such a rate that the amount of the active compound was 0.1 g/$m^2$. Therefore, without applying the emulsion, the plants were grown for 3 weeks. The results are shown in Table 11.

EXAMPLE 134

The emulsifiable concentrate prepared in Formulation Example 5 was diluted and applied to plants in the same way as in Example 133. The results are shown in Table 11.

EXAMPLE 135

The emulsifiable concentrate prepared in Formulation Example 6 was diluted and applied to plants in the same way as in Example 133. The results are shown in Table 11.

COMPARATIVE EXAMPLE 1

Formulations containing N-(2,4-dichlorophenoxyacetyl)-D-asparagine, N-(2,4-cichlorophenoxyacetyl)-D

aspartic acid, N-(2,4-dichlorophenoxyacetyl)-p-aminobenzoic acid, N-(2,4-dichlorophenoxyacetyl)-p-aminosalicylic acid, 2,4-dichlorophenoxyacetamide, 2,4-dichlorophenoxyacetanilide, and 2-methyl-4-chloro-phenoxyacetanilide respectively were prepared in accordance with Formulation Example 1. Each of the formulations was applied to plants in the same way as in Examples 1-48 so that the amount of each compound became 0.1 $g/m^2$. The results are shown in Table 12.

COMPARATIVE EXAMPLE 2

Formulations containing N-(2,4-dichlorophenoxyacetyl)-D-asparagine, N-(2,4-dichlorophenoxyacetyl)-D-aspartic acid, N-(2,4-dichlorophenoxyacetyl)-p-aminosalicylic acid, 2,4-dichlorophenoxyacetamide, sodium N-[2-(2,4-dichlorophenoxy)-propionyl]-L-methionine and sodium N-[2-(2,4-dichlorophenoxy)-propionyl]-L-leucine respectively were prepared in accordance with Formulation Example 2. Eech of the formulations was applied to plants in the same way as in Examples 49-91 so that the total amount of each of the compounds applied became 0.1 $g/m^2$. The results are shown in Table 13.

COMPARATIVE EXAMPLE 3

Formulations containing N-(2,4-dichlorophenoxyacetyl)-p-aminobenzoic acid, 2,4-dichlorophenoxyacetanilide, 2-methyl-4-chloro-phenoxyacetanilide and sodium N-[2-(2,4-dichlorophenoxy)-propionyl]-D,L-phenylalanine were prepared in accordance with Formulation Example 2. Each of the formulations was applied to plants in the same way as in Examples 92-101 so that the total amount of each of the compounds became about 0.1 $g/m^2$. The results are shown in Table 14.

Table 2

| Ex. No. | Plant / Compound No. | ① | ② | ③ | ④ | ⑤ | ⑥ | ⑦ | ⑧ | ⑨ | ⑩ | ⑪ | ⑲ |
|---|---|---|---|---|---|---|---|---|---|---|---|---|---|
| 1 | 1 | 4 | 5 | 5 | 5 | 5 | 5 | 5 | 4 | 3 | 1 | 5 | 0 |
| 2 | 2 | 5 | 5 | 5 | 5 | 5 | 5 | 5 | 4 | 5 | 1 | 4 | 0 |
| 3 | 3 | 1 | 5 | 5 | 5 | 5 | 5 | 5 | 4 | 5 | 1 | 4 | 0 |
| 4 | 4 | 1 | 5 | 5 | 5 | 1 | 5 | 4 | 3 | 2 | 1 | 1 | 0 |
| 5 | 5 | | | | 5 | | | 5 | 4 | 2 | 1 | 4 | 0 |
| 6 | 6 | | | | 5 | | | 5 | 5 | 3 | 3 | 3 | 0 |
| 7 | 7 | | | | 5 | | | 5 | 4 | 5 | 1 | 4 | 0 |
| 8 | 8 | 1 | 5 | 5 | 5 | 2 | 5 | 5 | 4 | 5 | 1 | 3 | 0 |
| 9 | 9 | | | | 5 | | | 5 | 4 | 4 | 1 | 2 | 0 |
| 10 | 10 | | | | 5 | | | 5 | 4 | 4 | 1 | 3 | 0 |
| 11 | 11 | | | | 5 | | | 4 | 3 | 3 | 2 | 1 | 0 |
| 12 | 12 | | | | 5 | | | 4 | 3 | 2 | 1 | 1 | 0 |
| 13 | 16 | 5 | 5 | 5 | 5 | 5 | 5 | 5 | 4 | 4 | 1 | 4 | 0 |
| 14 | 17 | 3 | 5 | 5 | 5 | 5 | 5 | 5 | 4 | 5 | 1 | 2 | 0 |
| 15 | 29 | 2 | 5 | 5 | 5 | 5 | 5 | 5 | 4 | 3 | 1 | 2 | 0 |

- to be continued -

Table 2 (continued)

| Ex. No. | Plant / Compound No. | ① | ② | ③ | ④ | ⑤ | ⑥ | ⑦ | ⑧ | ⑨ | ⑩ | ⑪ | ⑲ |
|---|---|---|---|---|---|---|---|---|---|---|---|---|---|
| 16 | 18 | | | | 5 | | | 5 | 4 | 5 | 1 | 2 | 0 |
| 17 | 20 | | | | 5 | | | 4 | 3 | 3 | 1 | 1 | 0 |
| 18 | 23 | 3 | 5 | 5 | 5 | 5 | 5 | 5 | 5 | 3 | 1 | 5 | 0 |
| 19 | 24 | 1 | 5 | 5 | 5 | 5 | 5 | 5 | 5 | 4 | 1 | 4 | 0 |
| 20 | 25 | 1 | 5 | 5 | 5 | 5 | 5 | 5 | 4 | 4 | 1 | 4 | 0 |
| 21 | 26 | 1 | 5 | 5 | 5 | 5 | 5 | 5 | 3 | 5 | 1 | 5 | 0 |
| 22 | 27 | 3 | 5 | 5 | 5 | 1 | 5 | 5 | 4 | 4 | 1 | 5 | 0 |
| 23 | 29 | | | | 4 | | | 4 | 3 | 2 | 0 | 0 | 0 |
| 24 | 43 | | | | 5 | | | 5 | 5 | 3 | 1 | 4 | 0 |
| 25 | 44 | | | | 5 | | | 5 | 5 | 4 | 3 | 1 | 0 |
| 26 | 47 | | | | 5 | | | 4 | 4 | 5 | 1 | 1 | 0 |
| 27 | 52 | | | | 5 | | | 5 | 4 | 2 | 2 | 1 | 0 |
| 28 | 53 | | | | 5 | | | 5 | 3 | 5 | 2 | 1 | 0 |
| 29 | 54 | | | | 5 | | | 4 | 3 | 2 | 1 | 1 | 0 |
| 30 | 56 | | | | 5 | | | 4 | 4 | 2 | 1 | 1 | 0 |

- to be continued -

Table 2 (continued)

| Ex. No. | Plant / Compound No. | ① | ② | ③ | ④ | ⑤ | ⑥ | ⑦ | ⑧ | ⑨ | ⑩ | ⑪ | ⑲ |
|---|---|---|---|---|---|---|---|---|---|---|---|---|---|
| 31 | 57 | | | | 5 | | | 4 | 3 | 3 | 1 | 1 | 0 |
| 32 | 67 | 2 | 5 | 5 | 5 | 3 | 5 | 5 | 5 | 3 | 1 | 1 | 0 |
| 33 | 68 | 3 | 5 | 5 | 5 | 1 | 5 | 5 | 3 | 2 | 2 | 1 | 0 |
| 34 | 31 | | | | 5 | | | 5 | 4 | 3 | 1 | 1 | 0 |
| 35 | 32 | | | | 5 | | | 5 | 4 | 3 | 1 | 1 | 0 |
| 36 | 33 | | | | 5 | | | 5 | 5 | 4 | 2 | 3 | 0 |
| 37 | 34 | | | | 5 | | | 4 | 4 | 2 | 1 | 1 | 0 |
| 38 | 35 | | | | 5 | | | 5 | 4 | 2 | 0 | 0 | 0 |
| 39 | 36 | | | | 5 | | | 5 | 5 | 3 | 2 | 2 | 0 |
| 40 | 42 | | | | 5 | | | 4 | 2 | 2 | 1 | 0 | 0 |
| 41 | 72 | | | | 4 | | | 4 | 3 | 4 | 1 | 1 | 0 |
| 42 | 73 | | | | 4 | | | 4 | 4 | 4 | 1 | 1 | 0 |
| 43 | 30 | | | | 5 | | | 5 | 5 | 3 | 1 | 3 | 0 |
| 44 | 18 | | | | 5 | | | 5 | 4 | 1 | 1 | 1 | 0 |
| 45 | 77 | | | | 4 | | | 5 | 4 | 4 | 1 | 1 | 0 |
| 46 | 78 | | | | 4 | | | 5 | 4 | 5 | 2 | 1 | 0 |
| 47 | 79 | | | | 4 | | | 4 | 4 | 3 | 1 | 2 | 0 |
| 48 | 80 | | | | 4 | | | 5 | 4 | 5 | 1 | 1 | 0 |

Table 3

| Ex. No. | Plant / Compound No. | (10) | (7) | (4) | (17) | (8) | (19) |
|---|---|---|---|---|---|---|---|
| 49 | 1 | 4 | 5 | 5 | 4 | 5 | 0 |
| 50 | 2 | 4 | 5 | 5 | 4 | 5 | 0 |
| 51 | 4 | 2 | 5 | 5 | 2 | 5 | 0 |
| 52 | 5 | 2 | 5 | 5 | 3 | 4 | 0 |
| 53 | 7 | 2 | 5 | 5 | 2 | 5 | 0 |
| 54 | 8 | 2 | 5 | 5 | 2 | 4 | 0 |
| 55 | 10 | 2 | 5 | 5 | 2 | 4 | 0 |
| 56 | 13 | 2 | 5 | 5 | 2 | 4 | 0 |
| 57 | 14 | 4 | 5 | 5 | 2 | 5 | 0 |
| 58 | 21 | 5 | 5 | 5 | 2 | 4 | 0 |
| 59 | 31 | 4 | 5 | 5 | 2 | 5 | 0 |
| 60 | 32 | 4 | 5 | 5 | 4 | 4 | 0 |
| 61 | 33 | 5 | 5 | 5 | 2 | 5 | 0 |
| 62 | 34 | 2 | 5 | 5 | 2 | 5 | 0 |
| 63 | 35 | 4 | 4 | 5 | 2 | 5 | 0 |
| 64 | 36 | 4 | 5 | 4 | 3 | 4 | 0 |

- to be continued -

Table 3 (continued)

| Ex. No. | Plant / Compound No. | (10) | (7) | (4) | (17) | (8) | (19) |
|---|---|---|---|---|---|---|---|
| 65 | 37 | 2 | 5 | 5 | 2 | 4 | 0 |
| 66 | 38 | 2 | 5 | 4 | 3 | 4 | 0 |
| 67 | 39 | 2 | 4 | 5 | 2 | 5 | 0 |
| 68 | 40 | 2 | 4 | 5 | 2 | 5 | 0 |
| 69 | 41 | 2 | 4 | 5 | 2 | 5 | 0 |
| 70 | 42 | 2 | 4 | 5 | 2 | 4 | 0 |
| 71 | 22 | 2 | 5 | 5 | 2 | 5 | 0 |
| 72 | 23 | 5 | 5 | 5 | 2 | 4 | 0 |
| 73 | 24 | 5 | 5 | 5 | 4 | 5 | 0 |
| 74 | 27 | 5 | 5 | 5 | 2 | 5 | 0 |
| 75 | 44 | 2 | 5 | 5 | 4 | 5 | 0 |
| 76 | 45 | 4 | 5 | 5 | 2 | 5 | 0 |
| 77 | 46 | 2 | 5 | 5 | 2 | 5 | 0 |
| 78 | 52 | 2 | 5 | 5 | 2 | 4 | 0 |
| 79 | 53 | 2 | 5 | 5 | 2 | 4 | 0 |
| 80 | 54 | 2 | 4 | 5 | 2 | 5 | 0 |

- to be continued -

Table 3 (continued)

| Ex. No. | Plant / Compound No. | ⑩ | ⑦ | ④ | ⑰ | ⑧ | ⑲ |
|---|---|---|---|---|---|---|---|
| 81 | 56 | 2 | 5 | 5 | 2 | 5 | 0 |
| 82 | 58 | 2 | 5 | 5 | 2 | 5 | 0 |
| 83 | 72 | 5 | 5 | 5 | 3 | 5 | 0 |
| 84 | 73 | 4 | 5 | 4 | 2 | 5 | 1 |
| 85 | 74 | 4 | 5 | 5 | 2 | 5 | 0 |
| 86 | 75 | 5 | 5 | 5 | 2 | 4 | 0 |
| 87 | 16 | 5 | 5 | 5 | 3 | 5 | 0 |
| 88 | 17 | 4 | 5 | 5 | 3 | 5 | 0 |
| 89 | 29 | 4 | 5 | 5 | 2 | 5 | 0 |
| 90 | 81 | 5 | 5 | 5 | 3 | 4 | 1 |
| 91 | 82 | 3 | 5 | 3 | 2 | 5 | 0 |

Table 4

| Ex. No. | Plant / Compound No. | (12) | (13) | (14) | (15) | (16) | (17) | (18) |
|---|---|---|---|---|---|---|---|---|
| 92 | 3 | 4 | 3 | 4 | 1 | 4 | 3 | 0 |
| 93 | 4 | 5 | 5 | 5 | 1 | 4 | 3 | 0 |
| 94 | 12 | 5 | 5 | 5 | 5 | 4 | 2 | 0 |
| 95 | 15 | 4 | 5 | 5 | 4 | 4 | 3 | 0 |
| 96 | 20 | 5 | 5 | 5 | 5 | 5 | 3 | 0 |
| 97 | 25 | 5 | 5 | 5 | 4 | 5 | 4 | 0 |
| 98 | 52 | 2 | 4 | 5 | 0 | 3 | 3 | 0 |
| 99 | 53 | 5 | 3 | 5 | 0 | 4 | 3 | 0 |
| 100 | 54 | 5 | 4 | 5 | 0 | 4 | 3 | 0 |
| 101 | 83 | 4 | 4 | 4 | 3 | 4 | 3 | 0 |

Table 5

| Ex. No. | Plant / Compound No. | (12) | (13) | (14) | (15) | (16) | (17) | (18) |
|---|---|---|---|---|---|---|---|---|
| 102 | 4 | 5 | 5 | 5 | 2 | 5 | 5 | 0 |
| 103 | 25 | 5 | 4 | 5 | 2 | 5 | 4 | 0 |

Table 6

| Ex. No. | Plant / Compound No. | ① | ② | ③ | ④ | ⑤ | ⑥ | ⑦ | ⑧ | ⑨ | ⑩ | ⑪ | ⑲ |
|---|---|---|---|---|---|---|---|---|---|---|---|---|---|
| 104 | 1 | | | | 5 | | | 5 | 4 | 2 | 1 | 4 | 0 |
| 105 | 2 | | | | 5 | | | 5 | 4 | 4 | 1 | 4 | 0 |
| 106 | 8 | | | | 5 | | | 5 | 4 | 4 | 1 | 3 | 0 |
| 107 | 23 | | | | 5 | | | 5 | 4 | 3 | 1 | 4 | 0 |
| 108 | 24 | | | | 5 | | | 5 | 4 | 3 | 1 | 2 | 0 |
| 109 | 27 | | | | 5 | | | 5 | 3 | 4 | 1 | 3 | 0 |
| 110 | 44 | | | | 5 | | | 5 | 4 | 4 | 1 | 1 | 0 |
| 111 | 47 | | | | 5 | | | 2 | 3 | 3 | 1 | 1 | 0 |

Table 7

| Ex. No. | Plant / Compound No. | ① | ② | ③ | ④ | ⑤ | ⑥ | ⑦ | ⑧ | ⑨ | ⑩ | ⑪ | ⑲ |
|---|---|---|---|---|---|---|---|---|---|---|---|---|---|
| 112 | 1 | | | | 5 | | | 5 | 4 | 2 | 1 | 1 | 0 |
| 113 | 2 | | | | 5 | | | 5 | 3 | 2 | 1 | 1 | 0 |
| 114 | 8 | | | | 5 | | | 4 | 3 | 2 | 1 | 1 | 0 |
| 115 | 44 | | | | 5 | | | 5 | 3 | 1 | 1 | 1 | 0 |

Table 8

| Ex. No. | Plant / Compound No. | (10) | (7) | (4) | (17) | (8) | (19) |
|---|---|---|---|---|---|---|---|
| 116 | 1 | 3 | 5 | 5 | 3 | 4 | 0 |
| 117 | 2 | 4 | 5 | 5 | 3 | 5 | 0 |
| 118 | 8 | 1 | 5 | 5 | 1 | 4 | 0 |
| 119 | 23 | 3 | 5 | 5 | 1 | 4 | 0 |
| 120 | 24 | 4 | 5 | 5 | 4 | 5 | 0 |
| 121 | 27 | 1 | 5 | 5 | 1 | 4 | 0 |
| 122 | 44 | 1 | 5 | 5 | 1 | 5 | 0 |
| 123 | 70 | 3 | 5 | 5 | 3 | 4 | 0 |
| 124 | 30 | 3 | 5 | 5 | 3 | 4 | 0 |

Table 9

| Ex. No. | Plant / Compound No. | (10) | (7) | (4) | (17) | (8) | (19) |
|---|---|---|---|---|---|---|---|
| 125 | 1 | 2 | 5 | 5 | 2 | 3 | 0 |
| 126 | 2 | 4 | 5 | 5 | 3 | 4 | 0 |

Table 10

| Ex. No. | Plant / Compound No. | (12) | (13) | (14) | (15) | (16) | (17) | (18) |
|---|---|---|---|---|---|---|---|---|
| 127 | 12 | 4 | 4 | 3 | 1 | 3 | 2 | 0 |
| 128 | 20 | 5 | 4 | 4 | 3 | 4 | 3 | 0 |
| 129 | 25 | 5 | 4 | 5 | 1 | 4 | 4 | 0 |
| 130 | 26 | 5 | 4 | 5 | 1 | 4 | 4 | 0 |
| 131 | 28 | 5 | 4 | 4 | 1 | 4 | 3 | 0 |
| 132 | 49 | 5 | 4 | 5 | 1 | 5 | 3 | 0 |

Table 11

| Ex. No. | Plant / Compound No. | (2) | (3) | (6) | (8) | (9) | (19) |
|---|---|---|---|---|---|---|---|
| 133 | 16 | 5 | 5 | 5 | 5 | 5 | 0 |
| 134 | 17 | 5 | 5 | 5 | 4 | 5 | 0 |
| 135 | 29 | 5 | 5 | 5 | 5 | 4 | 0 |

Table 12

| Compound | Plant | | | | | | |
|---|---|---|---|---|---|---|---|
| | (4) | (7) | (8) | (9) | (10) | (11) | (19) |
| N-(2,4-dichlorophenoxyacetyl)-D-asparagine | 0 | 0 | 0 | 0 | 0 | 0 | 0 |
| N-(2,4-dichlorophenoxyacetyl)-D-aspartic acid | 0 | 0 | 0 | 0 | 0 | 0 | 0 |
| N-(2,4-dichlorophenoxyacetyl)-p-aminobenzoic acid | 4 | 3 | 3 | 2 | 0 | 0 | 1 |
| N-(2,4-dichlorophenoxyacetyl)-p-aminosalicyclic acid | 5 | 5 | 4 | 2 | 2 | 1 | 2 |
| 2,4-Dichlorophenoxyacetamide | 3 | 5 | 4 | 2 | 1 | 1 | 2 |
| 2,4-Dichlorophenoxyacetanilide | 4 | 5 | 4 | 1 | 1 | 0 | 2 |
| 2-Methyl-4-chlorophenoxyacetanilide | 4 | 4 | 2 | 1 | 0 | 0 | 2 |

- to be continued -

Table 12 (continued)

| | Plant | | | | | | |
|---|---|---|---|---|---|---|---|
| | (4) | (7) | (8) | (9) | (10) | (11) | (19) |
| N-[2-(2,4-dichlorophenoxy)-propionyl]-L-methionine | 3 | 2 | 1 | 2 | 1 | 0 | 2 |
| N-[2-(2,4-dichlorophenoxy)-propionyl]-L-leucine | 3 | 3 | 1 | 2 | 1 | 0 | 2 |
| N-[2-(2,4-dichlorophenoxy)-propionyl]-D,L-phenylalanine | 3 | 2 | 2 | 1 | 1 | 0 | 2 |

Table 13

| Compound | Plant | | | | | |
|---|---|---|---|---|---|---|
| | ⑩ | ⑦ | ④ | ⑰ | ⑧ | ⑲ |
| N-(2,4-dichlorophenoxyacetyl)-D-asparagine | 0 | 2 | 0 | 0 | 0 | 0 |
| N-(2,4-dichlorophenoxyacetyl)-D-aspartic acid | 0 | 3 | 0 | 0 | 0 | 0 |
| N-(2,4-dichlorophenoxyacetyl)-p-aminosalicyclic acid | 2 | 5 | 5 | 2 | 4 | 3 |
| 2,4-Dichlorophenoxyacetamide | 1 | 5 | 3 | 1 | 4 | 2 |

Table 14

| Compound | Plant | | | | | | |
|---|---|---|---|---|---|---|---|
| | ⑫ | ⑬ | ⑭ | ⑮ | ⑯ | ⑰ | ⑱ |
| N-(2,4-dichlorophenoxyacetyl)-p-aminobenzoic acid | 2 | 3 | 3 | 0 | 4 | 2 | 2 |
| 2,4-Dichlorophenoxyacetanilide | 3 | 2 | 4 | 0 | 4 | 2 | 2 |
| 2-Methyl-4-chlorophenoxyacetanilide | 4 | 3 | 4 | 0 | 3 | 3 | 2 |

What is claimed is:

1. a compound represented by the following general formula (1)

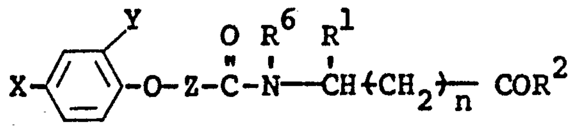 ... (1)

wherein

X is Cl or $CF_3$,

Y is Cl or $CH_3$,

Z represents a divalent saturated or unsaturated aliphatic hydrocarbon group having not more than 4 carbon atoms,

$R^1$ represents a group selected from the class consisting of a hydrogen atom, saturated aliphatic hydrocarbon groups having not more than 4 carbon atoms, a phenyl group, a benzyl group, a beta-methylthioethyl group, a hydroxymethyl group, an alpha-hydroxyethyl group, a hydroxyphenylmethyl group, a mercaptomethyl group and a benzylthiomethyl group,

$R^2$ represents the group $OR^3$ or the group $-N\begin{matrix}R^4\\R^5\end{matrix}$,

$R^3$ represents an aliphatic hydrocarbon group having not more than 15 carbon atoms which may be interrupted by an oxygen atom and may have a substituent, a phenyl group which may have a substituent or an ammonium cation,

$R^4$ and $R^5$ are identical or different and each represents a hydrogen atom, a saturated aliphatic hydrocarbon group having not more than 6 carbon atoms, or an alicyclic hydrocarbon group having 5 to 7 carbon atoms, or $R^4$ and $R^5$, taken

together with the nitrogen atom to which they are bonded, may form a 5- to 7-membered ring which may further contain a hetero atom,

$R^6$ represents a hydrogen atom or a saturated aliphatic hydrocarbon having not more than 4 carbon atoms or, taken together with $R^1$, may form $-CH_2CH_2CH_2-$ or

$-CH_2CH_2\overset{OH}{CH}-$, and

n is 0, 1 or 2.

2. The compound of claim 1 wherein $R^2$ in the formula represents the group $OR^3$ and $R^3$ represents an aliphatic hydrocarbon group having not more than 15 carbon atoms which may be interrupted by an oxygen atom and may have a substituent.

3. The compound of claim 1 wherein in the formula, $R^2$ is the group $OR^3$, and $R^3$ is an unsubstituted aliphatic hydrocarbon group having not more than 15 carbon atoms, an aliphatic hydrocarbon group having not more than 15 carbon atoms and a phenyl or hydroxyl group as the substituent, or an unsubstituted aliphatic hydrocarbon group having not more than 10 carbon atoms and being interrupted by an oxygen atom.

4. The compound of claim 1 wherein in the formula $R^2$ is the group $OR^3$, and $R^3$ is a phenyl group, or a phenyl group substituted by a halogen or an alkyl group having 1 to 5 carbon atoms.

5. The compound of claim 1 wherein Z in the formula is $-CH_2-$, $-\overset{CH_3}{CH}-$, $-CH_2CH_2-$, $-\overset{CH_3}{C}HCH_2-$, $-CH_2CH_2CH_2-$, $-\overset{CH_3}{C}HCH_2CH_2-$, $-CH_2\overset{CH_3}{C}HCH_2-$, $-\overset{CH_3}{CH}-\overset{CH_3}{CH}-$, $-\underset{CH_3}{\overset{CH_3}{C}}-CH_2-$, $-CH=CH-$, $-\overset{CH_3}{C}=CH-$, $-\overset{CH_2}{\overset{\|}{C}}-$, $-\overset{CH_2}{\overset{\|}{C}}-CH_2-$, $-CH_2-CH=CH-$ or $-CH=CH-CH=CH-$.

6. The compound of claim 1 wherein Z in the formula is $-CH_2-$, $-\overset{CH_3}{\overset{|}{C}}H-$ or $-CH_2CH_2CH_2-$.

7. The compound of claim 1 wherein $R^1$ in the formula is selected from the class consisting of hydrogen, methyl, ethyl, n-propyl, iso-propyl, n-butyl, iso-butyl, sec-butyl, tert-butyl, phenyl, benzyl, beta-methylthioethyl, hydroxymethyl, alpha-hydroxyethyl, hydroxyphenylmethyl, mercaptomethyl and benzylthiomethyl.

8. The compound of claim 1 wherein $R^1$ in the formula is hydrogen, methyl, ethyl, n-propyl, iso-propyl, n-butyl, iso-butyl, t-butyl, phenyl, benzyl or beta-methylthioethyl.

9. The compound of claim 1 wherein in the formula, $R^2$ is the group $-N\begin{matrix}R^4\\R^5\end{matrix}$, and $R^4$ and $R^5$ are identical or different and each represents hydrogen, methyl, ethyl, cyclopentyl, cyclohexyl or cycloheptyl.

10. The compound of claim 1 wherein $R^2$ is the group $-N\begin{matrix}R^4\\R^5\end{matrix}$ and $R^4$ and $R^5$, taken together, form $-(CH_2)_4-$, $-(CH_2)_5-$, $-(CH_2)_2O(CH_2)_2-$ or $-(CH_2)_2NH(CH_2)_2-$.

11. The compound of claim 1 wherein $R^6$ represents hydrogen, methyl, ethyl, n-propyl, iso-propyl, n-butyl, iso-butyl or tert-butyl, or taken together with $R^1$, forms $-CH_2CH_2CH_2$ or $-CH_2CH_2\overset{OH}{\overset{|}{C}}H-$.

12. The compound of claim 1 wherein n is 0.

13. A herbicide comprising as an active ingredient the compound of formula (1) defined in claim 1.

14. The herbicide of claim 13 wherein the active ingredient is a compound of the following formula

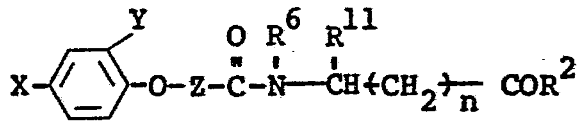 ... (1)-a

wherein X, Y, Z, $R^6$, $R^2$ and n are as defined in formula (1), and $R^{11}$ represents a hydrogen atom or a saturated aliphatic hydrocarbon group having not more than 4 carbon atoms.

15. The herbicide of claim 13 wherein the active ingredient is a compound represented by the formula

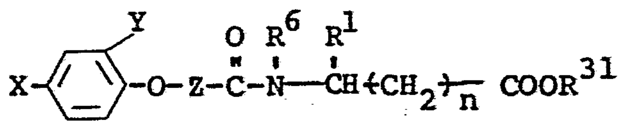 ... (1)-c

wherein X, Y, Z, $R^6$, $R^1$ and n are as defined in formula (1), and $R^{31}$ represents an aliphatic hydrocarbon group having not more than 15 carbon atoms which may be interrupted by an oxygen atom and may have a substituent.

16. The herbicide of claim 13 wherein the active ingredient is a compound represented by the following formula

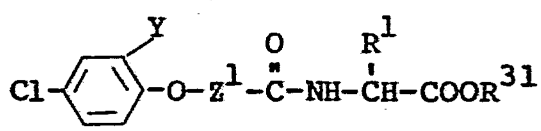 ... (1)-d

wherein Y and $R^1$ are as defined in formula (1), $R^{31}$ is as defined in formula (1)-c in claim 15, and $Z^1$ is $-CH_2-$ or $-CH(CH_3)-$.

17. The herbicide of claim 13 wherein the active ingredient is a compound of the following formula

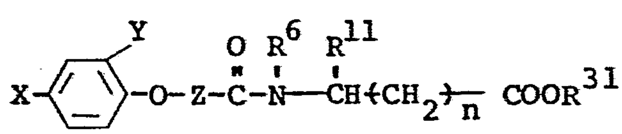 ... (1)-e

wherein X, Y, Z, $R^6$, $R^{11}$ and $R^{31}$ are as defined above.

18. The herbicide of claim 13 wherein the active ingredient is a compound of the following formula

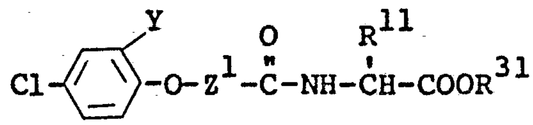 ... (1)-f

wherein X, $Z^1$, $R^{11}$ and $R^{31}$ are as defined above.

19. The herbicide of claim 13 wherein the active ingredient is a compound represented by the following formula

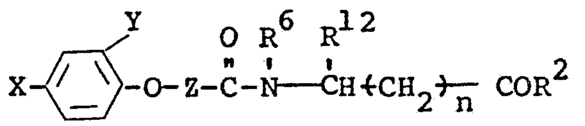 ... (1)-b

wherein X, Y, Z, $R^6$, $R^2$ and n are as defined in formula (1), and $R^{12}$ is a phenyl, benzyl or hydroxyphenyl group.

20. The herbicide of claim 13 wherein the active ingredient is a compound represented by the following formula

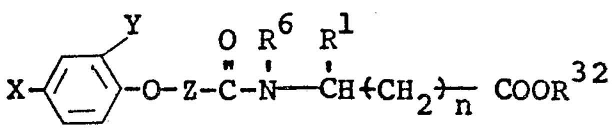 ... (1)-g

wherein X, Y, Z, $R^6$, $R^1$ and n are as defined above, and $R^{32}$ represents an aliphatic hydrocarbon group having not more than 15 carbon atoms which may be interrupted by an oxygen atom and may have a substituent, or a phenyl group which may have a substituent.

21. The herbicide of claim 13 wherein the active ingredient is a compound represented by the following formula

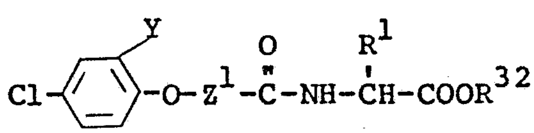 ... (1)-h

wherein Y, $Z^1$, $R^1$ and $R^{32}$ are as defined above.

22. The herbicide of claim 13 wherein the active ingredient is a compound represented by the following formula

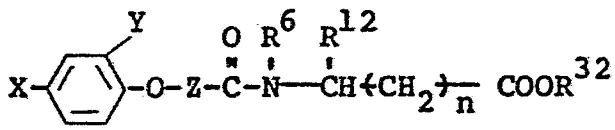 ... (1)-i

wherein X, Y, Z, $R^6$, $R^{12}$, $R^{32}$ and n are as defined in above.

23. The herbicide of claim 13 wherein the active ingredient is a compound represented by the following formula

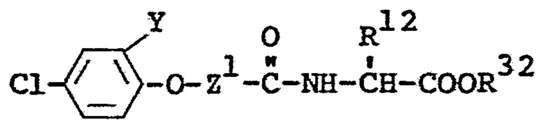 ... (1)-j

wherein Y, $Z^1$, $R^{12}$ and $R^{32}$ are as defined above.

24. The herbicide of claim 13 which further comprises a carrier and/or a surface-active agent.

25. A method for controlling weeds, which comprises applying a herbicidally sufficient amount of the compound of formula (1) defined in claim 1 to the seeds, stalks, leaves or roots of plants to be controlled or to the locus where said plants are growing or the growth of said plants is anticipated.

26. The method of claim 25 wherein the amount of the compound of formula (1) applied is 10g to 20kg per hectare.

27. The method of claim 25 wherein the amount of the compound (1) is an amount sufficient to inhibit the growth of, or eradicate, the plants.

28. The method of claim 25 wherein a herbicidally sufficient amount of the compound of formula (1) is applied to the locus where plants to be controlled or their seeds and a useful plant or its seeds grow together or are likely to grow together, whereby the plants to be conrolled or their seeds are selectively inhibited in growth or eradicated.

29. The method of claim 25 wherein the compound of formula (1) is applied to the locus in which rice or corn as a useful plant is growing or which is to be used for the growth of the useful plant, in an amount sufficient to control undesired plants which are growing, or are likely to grow, in said locus.

KRAUS · WEISERT & PARTNER

PATENTANWÄLTE
UND
ZUGELASSENE VERTRETER VOR DEM EUROPÄISCHEN PATENTAMT

0194403

Patentanwälte Thomas-Wimmer-Ring 15 D-8000 München 22

DR. WALTER KRAUS
DIPLOMCHEMIKER
DR.-ING. ANNEKÄTE WEISERT
DIPL.-ING.
JOHANNES SPIES
DIPL.-PHYS.

THOMAS-WIMMER-RING 15
D-8000 MÜNCHEN 22
TELEFON (0 89) 22 73 77
TELEX 5-212 156 kpat d
TELEFAX (0 89) 22 79 94

European Patent Office
Erhardtstr. 27

8000 Munich 2

EPA-EPO-OEB
DG 1
Reçu le
04 03 1986

UNSERE NR.:
OUR FILE: 5360/an
Bitte in der Antwort stets angeben
Please refer to in your reply

February 21, 1986

Re: European patent application 86 100 018.0
Teijin Limited

Enclosed are new pages 2, 3, 4, 8, 9, 10, 24, 43, 50, 65 and 67 (each in triplicate) which shall substitute the corresponding pages as originally filed. Applicant has found some typographical errors in the English text. In accordance with Rule 88 EPC, mistakes in documents filed with the European Patent Office may be corrected on request any time if the request for such a correction concerns a correction which is obvious in the sense that it is immediately evident that nothing else would have been intended than what is offered as the correction.

We also enclose copies of the corresponding pages as originally filed where we have indicated in handwriting the amendments carried out. It is politely requested to allow the corrections.

European Patent Attorney

Encls.: New pages and
correct. original pages
as mentioned above

KRAUS · WEISERT & PARTNER

PATENTANWÄLTE

UND

ZUGELASSENE VERTRETER VOR DEM EUROPÄISCHEN PATENTAMT





Patentanwälte Thomas-Wimmer-Ring 15 D-8000 München 22

DR. WALTER KRAUS
DIPLOMCHEMIKER

DR.-ING. ANNEKÄTE WEISERT
DIPL.-ING.

JOHANNES SPIES
DIPL.-PHYS.

THOMAS-WIMMER-RING 15
D-8000 MÜNCHEN 22
TELEFON (0 89) 22 73 77
TELEX 5-212 156 kpat d
TELEFAX (0 89) 22 79 94


European Patent Office
Erhardtstr. 27

8000 Munich 2

EPA-EPO-OEB
MÜNCHEN
Empfang bestätigt
Receipt acknowledged
Accuse reception
HH

EPA-EPO-OEB
DG 1
Reçu:
01 JUIL. 1986

UNSERE NR.: 5360 AW/an
OUR FILE:
Bitte in der Antwort stets angeben
Please refer to in your reply

June 19, 1986

Re: European patent application 86 100 018.0
Teijin Limited

Responsive to the telephone conversation we had with the Examiner, applicant submits the following:

With our brief dated February 21, 1986 new pages 2, 3, 4, 8, 9, 10, 24, 43, 50, 65 and 67 were filed at the European Patent Office. The following amendments had been carried out:

- Page 2, line 7: "(1954)" was changed to "(1945)";
- page 3, line 17: "methyl ester" was added after "DL-histidine";
- page 3, line 29: "chloroacetic acid" was changed to "chlorophenoxyacetic acid";
- page 4, line 18: "sugar beats" was changed to "sugar beets";
- page 8, line 26: "aliphatic" was added after "saturated";
- page 9, line 6: "sec-butyl" was added before "and";
- page 10, line 7: "a chlorine atom or" was added after "substituted by";


TELEGRAMMADRESSE: KRAUSPATENT POSTGIRO MÜNCHEN 851 02-809 BLZ 700 100 80
DEUTSCHE BANK MÜNCHEN 1 864 008 BLZ 700 700 10

page 24, lines 15: "corn fields" was changed to "an area where rice is cultivated";

page 43: the following data for compound No. 76 were added between the rows of compounds Nos. 75 and 77:

| | | | |
|---|---|---|---|
| | 3400 | $CDCl_3$ | 1.7 (3H) |
| | 1740 | | 3.8 (3H) |
| 76 | 1660 | | 4.1 (2H) |
| | 1540 | | 4.5-4.9 (1H) |
| | | | 7.0-7.4 (4H) |

;

page 43: "$C_2O$", the solvent for compound No. 77, was changed to "$D_2O$";

page 50, line 4: "and 2-methyl-4-chlorophenoxyacetanilide" was changed to "2-methyl-4-chlorophenoxyacetanilide, N-[2-(2,4-dichlorophenoxy)propionyl]-L-methionine, N-[2-(2,4-dichlorophenoxy)propionyl]-L-leucine and N-[2-(2,4-dichlorophenoxy)propionyl]-D,L-phenylalanine";

page 50, line 4: "and" was added after "salicyclic acid" and the comma before "sodium" was deleted;

page 50, lines 14 to 16: "sodium N-.....(omitted).....-L-leucine" was deleted;

page 50, lines 25 and 26: were entirely deleted and "anilide and 2-methyl-4-chlorophenoxyacetanilide" was substituted;

page 65, lines 21 and page 67, lines 13 and 17: $-N\begin{array}{l}R^4\\R^5\end{array}$ was changed to $-N\begin{array}{l}\diagup R^4\\\diagdown R^5\end{array}$ ;

page 67, line 11: "sec-butyl" was added after "isobutyl,".

European Patent Attorney

European Patent Office

# EUROPEAN SEARCH REPORT

0194403

Application number

EP 86 10 0018

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl.4) |
| X | GB-A-1 041 982 (BOOTS PURE DRUG CO.)<br>* Claims; page 6 * | 1,13-29 | C 07 C 103/46<br>C 07 C 103/66<br>C 07 D 207/16<br>C 07 D 295/18<br>C 07 C 149/243<br>A 01 N 39/02<br>A 01 N 43/36 |
| X | DE-A-1 927 692 (BEECHAM)<br>* Claims; examples 56-57 * | 1 | |
| X | CHEMICAL ABSTRACTS, vol. 62, no. 13, 21st June 1964, no. 16654h, Columbus, Ohio, US; N.B. MAHISHI et al.: "Chlorophenoxyacetic acids and their derivatives as possible tuberculostats", & J. INDIAN CHEM. SOC. 42(2), 67-74, 1965 | 1,13-29 | |
| | | | TECHNICAL FIELDS SEARCHED (Int. Cl.4) |
| Y | FR-A-1 458 106 (MERCK)<br>* Abstract * | 1 | |
| D,Y | FR-A-1 544 786 (L. NOUVEL)<br>* Abstract * | 1,13-29 | C 07 C 103/00<br>C 07 D 207/00<br>C 07 D 295/00<br>A 01 N 39/00<br>A 01 N 43/00 |
| D,Y | US-A-2 734 816 (J.W. WOOD et al.)<br>* Claims * | 1,13-29 | |

The present search report has been drawn up for all claims

| Place of search | Date of completion of the search | Examiner |
|---|---|---|
| THE HAGUE | 10-04-1986 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document